# EUROPEAN PATENT APPLICATION

(11) **EP 2 386 627 A1**
(43) Date of publication of application: **16.11.2011**
(21) Application number: 10004938.6
(22) Date of filing: 10.05.2010
(51) Int. Cl.: C12N 5/074

(54) **Induction of a mature hepatocyte phenotype**

(71) Applicant: Medizinische Hochschule Hannover, 30625 Hannover (DE)
(72) Inventor: Ott, Michael, 31515 Wunstorf (DE)
(74) Representative: Vossius & Partner

(57) **Abstract**

The present invention relates to a method for producing cells having a mature hepatocyte phenotype, comprising (a) providing a cell population comprising precursor cells of mature hepatocytes, wherein said cell population is obtained from a subject or derived from a cell line; and (b) introducing into said precursor cells a group of differentiation factors and/or the nucleic acid sequences encoding said differentiation factors, said group consisting of (i) one or more member(s) of the Foxa subfamily, (ii) HNF-4α and (iii) C/EBPα, thereby differentiating said precursor cells into cells having a mature hepatocyte phenotype.

## Description

The present invention relates to a method for producing cells having a mature hepatocyte phenotype, comprising (a) providing a cell population comprising precursor cells of mature hepatocytes, wherein said cell population is obtained from a subject or derived from a cell line; and (b) introducing into said precursor cells a group of differentiation factors and/or the nucleic acid sequences encoding said differentiation factors, said group consisting of (i) one or more member(s) of the Foxa subfamily, (ii) HNF-4α and (iii) C/EBPα, thereby differentiating said precursor cells into cells having a mature hepatocyte phenotype..

In this specification, a number of documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

In recent years the new concept of programmed cell fate change and differentiation through concerted expression of transcription factors has been emerged. This concept bypasses the state of pluripotency and instead directly induces the desired phenotype by transferring and expressing multiple transcription factors which were shown to change gene expression patterns and to induce cell fate changes in embryonic and adult stem/progenitor cells.

Takahashi and Yamanaka (Takahashi et al., 2006, 2007) were the first to show that the expression of a particular combination of transcription factors can induce a cell fate change and reprogram adult cells into embryonic stem cell like cells. In more detail, Takahashi and Yamanaka set a new paradigm with their experimental approach to reprogram mouse embryonic/adult fibroblasts to ES-like stem cells, referred to as induced pluripotent (iPS) cells, by retroviral transfer of the transcription factors Oct4, Sox2, c-Myc and Klf4. Although the differentiation potential of iPS cells is variable, meanwhile cells from all three germ layers including functional hepatocyte-like cells have been generated with protocols similar to those applied for ES cells. Using a similar approach Vierbuchen (Vierbuchen et al., 2010) transferred a set of transcription factors to directly induce a neuro-phenotype in fibroblasts.

However, to the best knowledge of the inventors so far no study has attempted multiple ectopic transcription factor expression for the induction of a mature hepatic phenotype. Currently various protocols for directed hepatic differentiation of stem cells have been developed in the prior art as alternatives for large scale hepatocyte production. For instance, embryonic stem cells can be grown in unlimited numbers and differentiate into functional hepatocyte-like cells in culture. Maintenance, growth and differentiation, however, are time consuming and expensive. Differentiation of cells takes up to 40 days and result in a cell population of cells with variable degrees of differentiation. Further, embryonic stem cells, for now, are not considered for cell therapy applications due to risks for teratoma formation and ethical concerns. In recent years, evidence has been provided that also adult cells from various sources (bone marrow/adipose tissue/placenta/umbilical cord) could occasionally overcome lineage borders and differentiate into hepatic lineage cells upon coordinated *in vitro* stimulation (Sgodda M, et al. (2007), Yamazaki S et al., (2003), Kang XQ,et al. 2005. Ong SY et al (2006)). Despite remarkable progress, the resulting cells often fail to achieve complete function sufficient for regenerative therapy and toxicology assays or other pharmacological assays, remaining only, "hepatocyte-like".

Therefore, mature hepatocytes are still frequently needed for *in vitro* toxicology or pharmaceutical assays and also potentially for cell therapies in patients with acute and metabolic liver diseases. However, liver tissues for hepatocyte isolation are rarely available. A further drawback is that hepatocytes show limited proliferation potential and dedifferentiate over time in culture. Thus, the isolation of high quality hepatocytes from organs and tissues for toxicological studies and clinical therapies remains a challenge and methods to obtain alternative cellular resources are urgently needed.

This need is addressed by the provision of the embodiments characterized in the claims.

Accordingly, in a first embodiment the invention relates to a method for producing cells having a mature hepatocyte phenotype, comprising (a) providing a cell population comprising precursor cells of mature hepatocytes, wherein said cell population is obtained from a subject or derived from a cell line; and (b) introducing into said precursor cells a group of differentiation factors and/or the nucleic acid sequences encoding said differentiation factors, said group consisting of (i) one or more member(s) of the Foxa subfamily, (ii) HNF-4α and (iii) C/EBPα, thereby differentiating said precursor cells into cells having a mature hepatocyte phenotype.

In accordance with the invention the term "precursor cell" refers to a cell having the capability to differentiate into a mature cell. Thus, a precursor cell specifies a cell which is partially or fully undifferentiated. With regard to the present invention, the precursor cell is a partially differentiated cell or a fully undifferentiated cell and has the capability to differentiate into a cell having a mature hepatocyte phenotype.

The term "mature hepatocyte" as used herein specifies the differentiated parenchymal cell of the liver. Hepatocytes make up 70-80% of the liver's cytoplasmic mass and are the predominant cell type in the liver. Hepatocytes are cells, for example characterized by the secretion of serum albumin, fibrinogen, and the prothrombin group of clotting factors (except for Factor 3 and 4). Moreover, hepatocytes are the main sites for the synthesis of lipoproteins, ceruloplasmin, transferrin, complement, and glycoproteins, nutrient metabolism and xenobiotic transformation.

The term "subject" as used herein means a vertebrate, preferably a mammal, more preferably any one of cat, dog, horse, cattle, swine, goat, sheep, mouse, rat, monkey, ape and human, and most preferably a human.

The term "differentiation factor" in accordance with the invention specifies any compound which when contacted with one or more precursor cell(s) leads to a more differentiated cellular stage, either when used alone or in combination with other differentiation factor(s). The term "transcription factor" also used herein specifies a specific subclass of the umbrella term "differentiation factors". To explain further, a transcription factor specifies a protein that binds to specific DNA sequences and thereby controls the transcription of genetic information from DNA to mRNA. In turn, the protein encoded by the mRNA leads to a more differentiated cellular stage. Thus, a transcription factor falls within the definition of a differentiation factor.

The term "introducing" as used herein specifies the process of bringing a protein and/or nucleic acids sequence into a living cell, an in particular also into the cell nucleus, preferably by introducing means as defined herein below. In accordance with the invention, protein to be introduced is (i) one or more member(s) of the Foxa subfamily, (ii) HNF-4α and (iii) C/EBPα and the nucleic acid sequences is the nucleic acid sequence encoding said differentiation factors

The term "Foxa familiy" specifies the subfamily a of Forkhead box transcription factors. The Foxa familiy comprises Foxa1 (human DNA/protein SEQ ID NOs: 1 and 2; mouse DNA/protein SEQ ID NOs. 11 and 12), Foxa2 (human DNA/protein SEQ ID NOs: 3 and 4; mouse DNA/protein SEQ ID NOs. 13 and 14) and Foxa3 (human DNA/protein SEQ ID NOs: 5 and 6; mouse DNA/protein SEQ ID NOs. 15 and 16) (Kaestner et al. (2000), Genes Dev. 14(2):142-146). Also encompassed are fragments of Foxa as long as these fragments retain their differentiation effect on the precursor cells used in accordance with the invention.

The term "HNF-4α" specifies a nuclear receptor of the hepatocyte nulcear factor 4 subfamily that binds to DNA (human DNA/protein SEQ ID NOs: 7 and 8; mouse DNA/protein SEQ ID NOs. 17 and 18) (Chartier et al. (1994), Gene 147 (2): 269-72 and Wisely et al. (2002), Structure 10 (9): 1225-34.). Also encompassed are fragments of HNF-4α as long as these fragments retain their differentiation effect on the precursor cells used in accordance with the invention.

The term "C/EBPα" refers to the α member of the CCAAT-enhancer-binding proteins (or C/EBPs) which are a family of transcription factors (human DNA/protein SEQ ID NOs: 9 and 10; mouse DNA/protein SEQ ID NOs. 19 and 20) (Ramji et al. (2002), Biochem. J. 365:561-575 and Kovács et al (2003), J Biol Chem., 278(38):36959-65). Also encompassed are fragments of C/EBPα as long as these fragments retain their differentiation effect on the precursor cells used in accordance with the invention.

The term "nucleic acid molecule" is defined herein below.

The method of the invention, in particular step (b), is carried out under suitable cell culture conditions. It is of note that cell culture conditions for a cell population comprising precursor cells of mature hepatocytes obtained from a subject or derived from a cell line are well known in the art (e.g. Cooper GM (2000). "Tools of Cell Biology", ISBN 0-87893-106-6; K. Turksen, ed., Humana Press, 2004, "Adult stem cells" ISBN-10 1-58829152-9, J. Masters, ed., Oxford University Press, 2000, "Animal cell culture", ISBN-10 0-19-963796-2). Suitable culture conditions are for example shown in more detail in the Examples of the invention (e.g. the liver differentiation media LD1 and LD 2).

The differentiation factors consisting of (i) one or more member(s) of the Foxa subfamily, (ii) HNF-4α and (iii) C/EBPα are either introduced simultaneously or sequentially in the method of the invention. In this regard it is preferred that the factor according to (i) is introduced before using, e.g. adding factors according to (ii) and (iii), and even more preferred that first the factor according to (i), second the factor according to (ii) and third the factor according to (iii) is introduced, e.g. added. In this regard the term "introduced" also embraces the term "expressed".

Induction and maintenance of hepatocyte differentiation and control of liver-specific gene expression is attributed to a large extent to hepatocyte nuclear factors (HNFs) (Kyrmizi et al., 2006, Lemaigre FP,2009). This class of proteins includes five families of transcriptional regulators: HNF1, Foxa (formerly HNF3), C/EBP, HNF4, and HNF6. While none of these factors is exclusively expressed in the liver, the combinatorial actions of tissue-specific transcription factors collaborate to achieve the stringency and dynamic regulation of gene expression required for the proper development and function of the organ. The vertebrate Foxa subfamily is closely related to the Drosophila protein FORK HEAD and comprises *Foxa1, Foxa2* and *Foxa3*. Foxa proteins function as transcriptions factors, as defined by the presence of sequence-specific DNA-binding activity and the ability to regulate transcription of target genes. Foxa proteins are required for normal development of endoderm derived organs such as the liver, pancreas, lungs and prostate. Recent studies have provided evidence for the hypothesis that Foxa proteins act as "competence factors" and facilitate binding and transcriptional activity of other transcription factors in endoderm tissues and cells. Moreover it has been shown that Foxa2 is required for normal liver homeostasis also in the adult liver, as >43% of genes expressed in the liver were also associated with Foxa2 binding (Wederell E 2008).
The hepatic nuclear receptor HNF4α is a key regulator of both hepatocyte differentiation during embryonic development and maintenance of a differentiated phenotype in the adult (Zaret K and Grompe M, 2008 Hayhurst et al., 2008, Parviz F., et al. 2003, Li J et al. 2000). Recent studies using mouse embryos, in which the *Hnf4* gene is ablated specifically from fetal hepatocytes, have shown that Hnf4α is essential for the generation of a hepatic epithelium. Furthermore, in the same study, it was demonstrated that HNF4α could induce a mesenchymal to epithelial transition when expressed in naive NIH 3T3 fibroblasts.
Similarly, expression of C/EBPα is critically important for maintaining the differentiated state of hepatocytes in vivo. It is downregulated in rat liver nodules and HCCs and forced expression of C/EBPα was found to impair proliferation and suppress tumorigenicity (Nerlov C., 2007, Tseng HH et. al, 2009). In adult mice, conditional knockout experiments have shown that C/EBPa plays an important role in hepatic glucose, nitrogen, bile acid and iron metabolism all of which represent highly differentiated hepatocyte functions (Tan et al. (2007)).

The inventors have surprisingly found that only three distinct transcription factors, namely (i) one or more member(s) of the Foxa subfamily, (ii) HNF-4α and (iii) C/EBPα proteins selected from the members of the five families of transcriptional regulators for hepatocyte differenciation, namely HNF1, Foxa, C/EBP, HNF4, and HNF6 are essential for appropriate differentiation of precursor cells into cells having a hepatocyte phenotype. In more detail, in the examples provided herein the ability to induce a mature hepatocyte phenotype, for example in clonally expanded adult liver derived progenitor cells (ALDP's) by sequential lentiviral over-expression of this set of liver enriched transcription factors, namely Foxa2, HNF4α and C/EBPα is shown.

The method provided in accordance with the invention allow for large scale hepatocyte production. Moreover, the method of the invention is, to the best knowledge of the inventors, a more rapid (7 days as shown in the appended examples) and efficient differentiation approach as compared to the methods known in the art.

As known in the art, induction and maturation of a hepatocyte phenotype in the embryonic liver is governed by the orderly and hierarchical expression of liver enriched transcription factors. As explained in more detail herein above, for example the hepatic nuclear factors and CCAT/Enhancer binding proteins play key roles in the developing liver as well as in the regulation of liver specific gene expression in the adult organ. However, whereas the inventors do not wish to be bound by any theory, it is hypothesized that over-expression of members of these transcription factor families could induce and maintain a mature hepatic phenotype in a liver progenitor cell line, it was not known which transcription factors or more general which differentiation factors are essential and sufficient to induce and maintain a mature hepatic phenotype. As shown in the examples, the three transcription factors, namely Fox2a, HNF-4α and C/EBPα expressed in ALDP cells are essential. These factors were selected based on known functions in liver development and most importantly based on the following criteria set and investigated by the inventors: (1) > 10 fold up-regulation during in mouse embryonic liver compared to whole embryo tissue, (2) >10 fold downregulation in cultured hepatocytes over a culture period of six days and (3) >3 fold induction in embryonic stem cells throughout the hepatic differentiation process. Applying these innovative criteria led to the identification of Fox2a, HNF-4α and C/EBPα and the unexpected finding that the morphological and metabolic phenotype of triple transduced ALDP cells surprisingly closely resemble mature mouse hepatocytes.

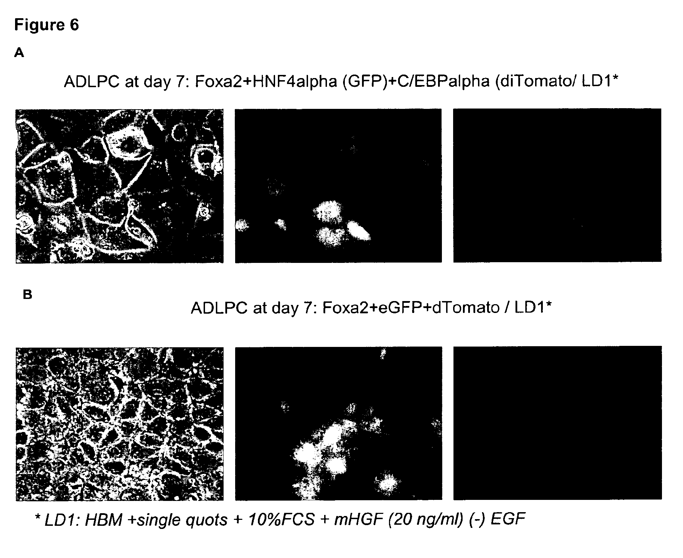

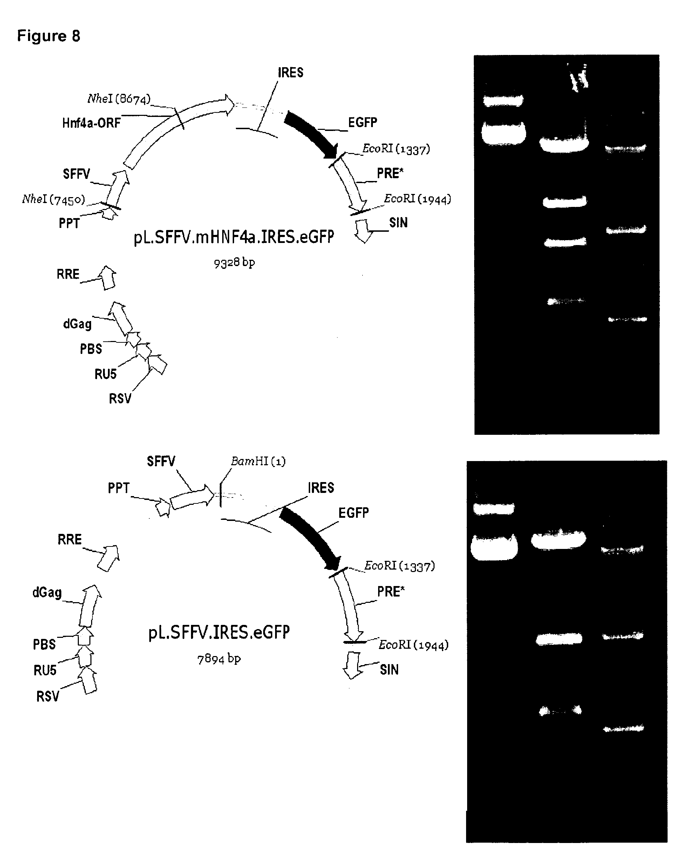

The mature mouse hepatocytes phenotype is evidenced by the fact that cultures treated with all three transcription factors contained significantly more cells with two or more nuclei, which is a hallmark of adult mouse hepatocytes. Moreover, an incremental increase in albumin secretion in single, double and triple transduced cells is observed. To the best knowledge of the inventors, it is for the first time reported that albumin secretion of differentiated hepatocyte-like cells is identical to adult hepatocytes (cf. Figure 8). PAS positive staining, which suggests glycogen storage ability, was also comparable to adult hepatocytes (cf. Figure 6). As previously reported, Foxa2 overexpression in mesenchymal stem cells generated only marginal PAS positivity when compared to HUH7 controls (Ishii et al. (2008). Ureagenesis was also maximally induced in triple transduced cells, however at a much lower level compared to day 1 cultured hepatocytes. Although the expression of carbamoylphosphate synthase-1 (CPS-1), a rate-limiting enzyme in urea synthesis, was reported to be strongly decreased in the absence of C/EBPα (Inoue et al. (2004)), the over-expression of this transcription factor in the experimental setting does not seem to be enough to bring ureagenesis in the hepatocyte range. Over-expression of C/EBPα even induces toxic effects in ALDP cells and fibroblast cultures in the absence of HNF4α overexpression and this effect seems to be dose and time-dependent . This finding is consistent with previous reports that ectopic expression of C/EBPα gene could induce hepatic stellate cells apoptosis in a dose-and time-dependent manner as shown by Annexin V/Pl staining, caspase-3 activation assay, and flow cytometry (Wang et al. (2009)). It further suggests that optimal effects of CEBPα which lead to terminal hepatic differentiation require fine tuning and can be achieved only in a predifferentiated hepatocyte-like state.

Although also prior art studies on hepatocyte differentiation report positive functional testing for hepatocyte characteristics, the positive controls used were not cultured mature adult hepatocytes. In other words, the efficiency of hepatic differentiation is difficult to appreciate in comparison with other cells than mature adult hepatocytes and most likely did not reach the level of mature hepatocytes as shown herein for the cells differentiated according to the method of the invention.

In more detail, in the prior art it is has only been shown that mouse ES cells were able to differentiate into hepatocyte-like cells with liver specific metabolic functions when Foxa2 transfected cells were grown in α-MEM supplemented with FGF2, nicotinamide dexamethasone, L-ascorbic-2-phosphate and 10% FBS in a 3 dimensional culture system to form spheroids (Ishikaza et al (2002)). In a recent study, stage specific forced expression of homeobox gene Hex in embryoid bodies from wild type ESCs led to the up-regulation of albumin and Afp expression and secretion of albumin and transferrin. Microarray analysis showed that Hex regulated the expression of a broad spectrum of hepatocyte-related genes, including fibrinogens, apolipoproteins, and cytochromes. The effects were restricted to c-kit(+) endoderm-enriched EB-derived populations, suggesting that Hex functions at the level of hepatic specification of endoderm (Kubo et al. (2010)). Ishii et al. loc. lit. have established a tetracycline-regulated expression system for Foxa2 in UE7T-13 BM-MSCs showing that expression of Foxa2 significantly enhanced expression of albumin, alphafetoprotein (AFP), tyrosine amino transferase (TAT) and epithelial cell adhesion molecule (EpCAM) genes. The differentiated cells showed hepatocyte-specific functions including glycogen production and urea secretion, however only when compared to the cell lines HUH-7 or HepG2 controls.

In conclusion, it is shown in accordance with the present invention that expression of Foxa2, HNF4α and C/EBPα induces a mature hepatocyte-like phenotype, for example in an expandable liver derived progenitor cell line leading to better results with regard to the analogy to mature hepatocytes as compared to conventional differentiation protocols. The excellent expansion capability of the ADLPCs used and the findings with regard to their capacity to differentiae to cells having a mature hepatocyte phenotype when applying the method of the invention makes them attractive hepatocyte precursor sources to be used for drug testing studies and as a basis for developing bioartificial liver support devices.

Accordingly the invention furthermore relates to a bioartifical liver support comprising the cells obtained by the method of the invention.

The term "bioartifical liver support" as used herein refers to a compound which may be used in the treatment of a liver disease as defined herein below.

In accordance with a preferred embodiment, the method of the invention further comprises: (c) isolating cells having a mature hepatocyte phenotype from the cell population obtained in step (b) above.

Methods for isolating cells are well known in the state of the art and comprise FACS (fluorescence activated cell sorting), sucrose gradient centrifugation, (laser) microdissection, single cell dilution, and Magnetic Labeled Bead Cell Separation. FACS is a method for sorting a heterogeneous mixture of biological cells into two or more containers, one cell at a time, based upon the specific light scattering and fluorescent characteristics of each cell. For this purpose a cell specific marker. e.g. a hepatocyte-specific cell-surface marker (for example the a hepatocyte-specific cell-surface marker cytokeratin 8 or 18) may be labeled with a fluorescent dye like FLAG, GFP, YFP, RFP, dTomato, cherry, Cy3, Cy 5, Cy 5.5., Cy 7, AMCA, Tamra, Texas Red, rhodamine, Alexa fluors, FITC or TRITC. Sucrose gradient centrifugation is a centrifugation technique which separates compounds according to their density along a sucrose density gradient which is typically created overlaying lower concentrations of sucrose on higher concentrations in a centrifuge tube. Single cell dilution is a technique of diluting cells, preferably in culture medium until a concentration is reached that allows to separate single cell in a separated vial. Magnetic Labeled Bead Cell Separation is a commercial system available from Miltenyi Biotec (MACS), Bergisch Gladbach, Germany.

In a more preferred embodiment the method of the invention the introduction into the precursor cells is effected by transforming precursor cells of the cell population of (a) with one or more nucleic acids molecule(s) comprising the nucleic acid sequences encoding (i) one or more member(s) of the Foxa subfamily, (ii) HNF-4α and (iii) C/EBPα in an expressible form.

The term "nucleic acid molecule", in accordance with the present invention, includes DNA, such as cDNA or genomic DNA, and RNA. It is understood that the term "RNA" as used herein comprises all forms of RNA including mRNA. The term "nucleic acid molecule" is interchangeably used in accordance with the invention with the term "polynucleotide".

The nucleic acid molecule(s) may also comprise regulatory regions or other untranslated regions. In a further embodiment, said nucleic acid molecule may comprise heterologous nucleic acid which may encode heterologous proteinaceous material thus giving rise, e.g., to fusion proteins.

The present invention thus relates to a the nucleic acid molecule(s) of the invention encoding a fusion protein. For example, the nucleic acid molecule of the invention can be fused in frame to a detectable marker such as purification marker or a direct or indirect fluorescence marker. Purification marker comprise one or more of the following tags: His, lacZ, GST, maltose-binding protein, NusA, BCCP, c-myc, CaM, FLAG, GFP, YFP, cherry, thioredoxin, poly(NANP), V5, Snap, HA, chitin-binding protein, Softag 1, Softag 3, Strep, or S-protein. Suitable direct or indirect fluorescence marker comprise FLAG, GFP, YFP, RFP, dTomato, cherry, Cy3, Cy 5, Cy 5.5., Cy 7, DNP, AMCA, Biotin, Digoxigenin, Tamra, Texas Red, rhodamine , Alexa fluors, FITC, TRITC or any other fluorescent dye or hapten.

Methods for transformation of cells are well established in the state of the art and include but are not limited to viral transformation (e.g. adenoviral, adeno-associated, retroviral, lentiviral transfection), transposon transformation (e.g. retrotransposons, DNA-transposons, retroviruses as transposable elements, and in particular Tc1/mariner-class transposons like piggyBac and Sleeping Beauty), lipofection, microinjection, electroporation, impalefection, gene gun, magnetofectin, sono-poration, optical transfection (e.g. by a laser), and chemical-based transfection.

In an even more preferred embodiment of the method of the invention the one or more nucleic acids molecule(s) are one or more vector(s).

Accordingly, the present invention also relates to a vector containing the nucleic acid molecule(s) of the present invention. Preferably, the vector is a plasmid, cosmid, virus, bacteriophage or another vector used e.g. conventionally in genetic engineering.

The nucleic acid molecule may be inserted into several commercially available vectors. The nucleic acid molecule(s) referred to above may also be inserted into vectors such that a translational fusion with another polynucleotide is generated. The other polynucleotide may encode a protein which may e.g. increase the solubility, correct protein folding, facilitate the purifcation of the cells of the invention and/or further differentiation factors enhancing differentiation and/or maintenance of the mature hepatocyte phenotype. For vector modification techniques, see Sambrook and Russel (2001), loc. cit. Generally, vectors can contain one or more origin of replication (ori) and inheritance systems for cloning or expression, one or more markers for selection in the host, e. g. dihydrofolate reductase, G418, neomycin, ampicillin, hygromycin, or kanamycin, and one or more expression cassettes. Suitable origins of replication (ori) include, for example, the Col E1, the SV40 viral and the M 13 origins of replication. The coding sequences inserted in the vector can e.g. be synthesized by standard methods, or isolated from natural sources. Ligation of the coding sequences to transcriptional regulatory elements and/or to other amino acid encoding sequences can be carried out using established methods. Transcriptional regulatory elements (parts of an expression cassette) ensuring expression in prokaryotes or eukaryotic cells are well known to those skilled in the art. These elements comprise regulatory sequences ensuring the initiation of transcription (e. g., translation initiation codon, promoters, such as naturally-associated or heterologous promoters and/or insulators), internal ribosomal entry sites (IRES) (Owens, Proc. Natl. Acad. Sci. USA 98 (2001), 1471-1476) and optionally poly-A signals ensuring termination of transcription and stabilization of the transcript. Additional regulatory elements may include transcriptional as well as translational enhancers.

An expression vector according to this invention is capable of directing the replication, and the expression, of the polynucleotide and encoded differentiation factor(s) of this invention. Suitable expression vectors which comprise the described regulatory elements are known in the art. The nucleic acid molecules as described herein above may be designed for direct introduction, phage vectors or viral vectors (e.g. adenoviral, retroviral) into a cell.

A typical mammalian expression vector contains the promoter element, which mediates the initiation of transcription of mRNA, the protein coding sequence, and signals required for the termination of transcription and polyadenylation of the transcript and moreover, elements such as origin of replication, drug resistance gene, regulators (as part of an inducible promoter) may also be included. Highly efficient transcription can be achieved with the early and late promoters from SV40, the long terminal repeats (LTRs) from retroviruses, e.g., RSV, HTLVI, HIVI, and the early promoter of the cytomegalovirus (CMV). Alternatively, the recombinant polypeptide can be expressed in stable cell lines that contain the gene construct integrated into a chromosome. The co-transfection with a selectable marker such as dhfr, gpt, neomycin, hygromycin allows the identification and isolation of the transfected cells. The transfected nucleic acid can also be amplified to express large amounts of the encoded polypeptide.

In an even more preferred embodiment of the method of the invention the one or more vector(s) are one or more lentiviral vector(s).

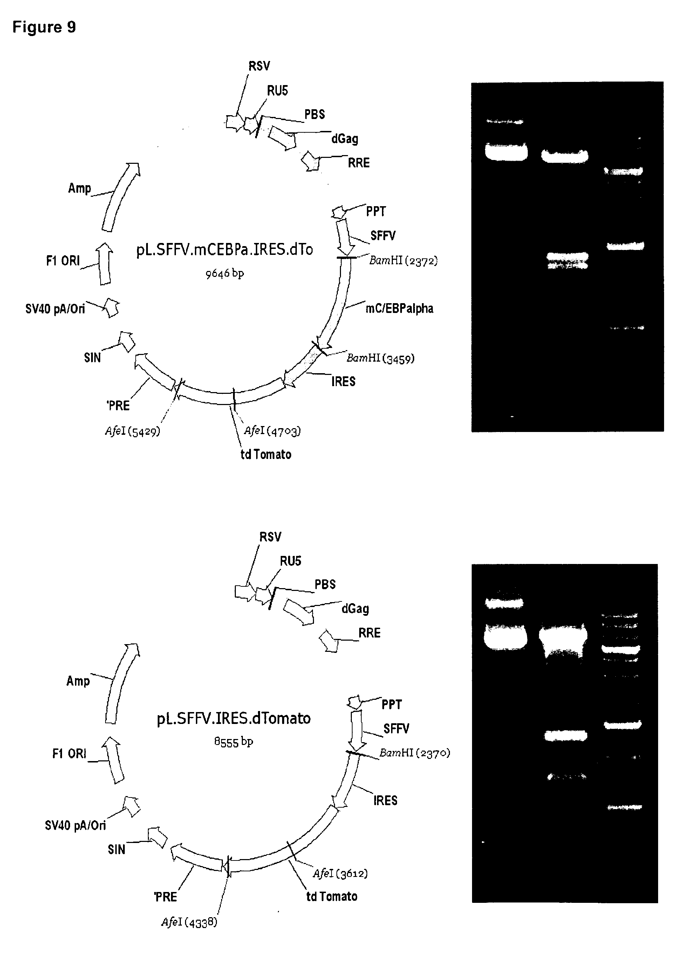

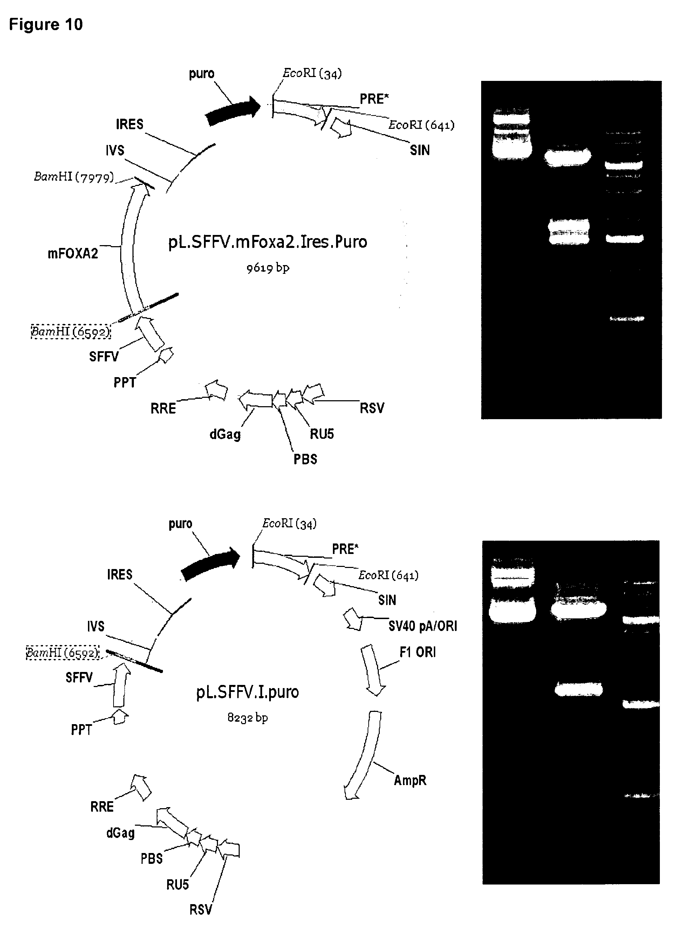

Examples of lentiviruses providing elements of a lentiviral vector are HIV, SIV, FIV, Puma lentivirus, EIA, Bovine immunodeficiency virus, Caprine arthritis encephalitis virus, or Visna/maedi virus. Preferably used in accordance with the invention is a 3^{rd} generation lentiviral expression plasmid containing a SFFV promoter driven IRES-Puro (Foxa2), IRES-dTomato (C/EBPα) or IRES-eGFP (HNF4α) expression cassette. More preferably, one or more vectors as shown in Figures 8 to 10 are used. Lentiviruses are particularly useful in the method for the invention because they integrate into the genome and thus allow for non-transient gene expression. Moreover, studies have shown that lentivirus vectors have a lower tendency to integrate in places that potentially cause cancer than gamma-retroviral vectors which also integrates into the genome (Cattoglio et al. (2007), Blood, 110(6): 1770-1778). Thus they have a lower tendency of abnormal cell growth upon integration.

In accordance with a more preferred embodiment of the method of the invention the nuclei acid sequences encode (i) the one or more member(s) of the Foxa subfamily, (ii) HNF-4α and (iii) C/EBPα in an inducible expressible form.

Systems for inducible expression are widely used in the state of the art and include but are not limited to the Tet-on system, Tet-off system, lac operator/expressor system and the commercial systems Q-mate™,Rheo Swith®, Cumate® or TREX™. As known in the art inducible expression systems allow to control the expression of a gene over time, the order of the genes expressed (provided that different expression systems, e.g. a Tet-on system and Tet-off system are used in parallel or subsequently for the expression of different genes) and also the amount of expressed mRNA from the vector.

In a further more preferred embodiment the method of the invention the introduction into the precursor cells is effected by microinjection, electroporation, lipofection and/or protein transduction of (i) one or more member(s) of the Foxa subfamily, (ii) HNF-4α and (iii) C/EBPα into said precursor cells.

This Methods for introducing a protein (or peptide) directly into a cell are well known in the state of the art and comprise but are not limited to microinjection, electroporation, lipofection (using liposomes) and protein transduction. In this regard, the proteins to be introduced may either be isolated form their natural environment or recombinantly produced. These methods are capable to bypass or further support the step of transforming a cell as described herein above.
A liposome used for lipofection is a small vesicle, composed of the same material as a cell membrane (i.e., normally a lipid bilayer e.g. out of phospholipids), which can be filled with one more protein(s) (e.g. Torchilin VP. (2006), Adv Drug Deliv Rev., 58(14):1532-55). To deliver a protein into a cell, the lipid bilayer of the liposome can fuse with the lipid bilayer of the cell membrane, thereby delivering the contained protein into the cell. It is preferred that the liposomes used in accordance with invention are composed of cationic lipids which. The cationic liposome strategy has been applied successfully to protein delivery (Zelphati et al. (2001). J. Biol. Chem. 276, 35103-35110). As known in the art, the exact composition and/or mixture of cationic lipids used can be altered, depending upon the protein(s) of interest and the cell type used (Felgner et al. (1994). J. Biol. Chem. 269, 2550-2561).
Protein transduction specifies the internalisation of proteins into the cell, from the external environment (Ford et al (2001), Gene Therapy, 8:1-4). This method relies on the inherent property of a small number of proteins and peptides (preferably 10 to 16 amino acids long) being able to penetrate the cell membrane. The transducing property of these molecules can be conferred upon proteins which are expressed as fusions with them and thus offers, for example, an alternative to gene therapy for the delivery of therapeutic proteins into target cells. Commonly used proteins or peptides being able to penetrate the cell membrane are, for example; the antennapedia peptide, the herpes simplex virus VP22 protein, HIV TAT protein transduction domain, peptides derived from neurotransmitters or hormones, or a 9xArg-tag.

Microinjection and electroporation are well known in the art and the skilled person knows how to perform these methods. Microinjection refers to the process of using a glass micropipette to introduce substances at a microscopic or borderline macroscopic level into a single living cell. Electroporation is a significant increase in the electrical conductivity and permeability of the cell plasma membrane caused by an externally applied electrical field. By increasing permeability protein (or peptides or nucleic acid sequences) can be introduced into the living cell.

In accordance with a preferred embodiment of the method of the invention the member of the Foxa subfamily is Foxa2.

The member Fox2a of the Foxa family has been used in the examples of the invention provided herein below. Thus, the use of Fox2a is preferred.

In another preferred embodiment of the method of the invention the precursor cells of mature hepatocytes are embryonic stem cells, adult stem cells, or iPS cells.

Stem cells like embryonic stem cells, adult stem cells, or iPS cells are characterized by their ability to renew themselves through mitotic cell division and differentiate into a diverse range of specialized cell types, including hepatocytes. Accordingly, embryonic stem cells, adult stem cells, or iPS cells are particularly suitable precursor cells in accordance with the invention.

The embryonic stem cells as far as being human embryonic stem cells were not established by the inventors by a process involving the step of disrupting a human embryo. In other words, the method of the invention is practised without the need of disrupting a human embryo, by for example using human embryonic stem cell lines established and available at the time the invention was made. Such cells are inter alia described in Thomson et. al (1998), "Blastocysts Embryonic Stem Cell Lines Derived from Human", Science 282 (1145): 1145-1147, or Thomson et al. (1998), "Embryonic stem cell lines derived from human blastocysts", Science 282 (5391): 1145-7. Specific examples are the cell lines CHB-1, CHB-2, or CHB-3.

In an alternative preferred embodiment of the method of the invention the precursor cells of mature hepatocytes are (a) primitive epithelial progenitor cells obtained from liver suspension, or (b) adult liver derived progenitor cells.

Primitive epithelial progenitor cells obtained from liver suspension, or adult liver derived progenitor cells are precursor cells of mature hepatocytes which already have partly differentiated into the lineage of mature hepatocytes but however - in contrast to fully differentiated mature hepatocytes - retained the capability to grow in vitro. Due to this advantage, they are particularly preferred precursor cells in accordance with the invention. Adult liver derived progenitor cells we also employed in the examples provided by the invention.

Methods to obtain primitive epithelial progenitor cells obtained from liver suspension, or adult liver derived progenitor cells are well established in the state of the art and described, for instance in the examples provided herein.

In more detail, the adult liver derived progenitor cells (ALDPs) can be obtained from adult liver cell suspension cultures in the presence of growth factor supplemented medium and were described as bipotent adult mouse liver (BAML) cells by others. Whether they exist in the liver or derive from hepatocytes or other cell types in the presence of growth factors in culture, is currently not known. The cells are expandable to huge numbers and theoretically can be generated even from a patient's liver biopsy. Further, abnormal cell growth, in particular the formation of a tumour from these cells was not reported in the literature. This makes these cells attractive for cell therapy applications. Proliferation capacity, expression of endoderm markers (Sox17, GATA4), liver enriched transcription factors, E- and N- Cadherins as well as cytokeratins 7, 8, 18, 19 and hepatic and biliary differentiation potential qualifies the cells as progenitors of mature liver cells. To the best knowledge of the inventors, so far attempts to differentiate ALPDs *in vitro* towards mature hepatocytes, by conventional differentiation factors, has resulted in only marginal induction of hepatic gene expression and hepatocyte functions. As shown in the examples provided herein, ALDPs can effectively be differentiated into cells having a mature hepatocyte phenotype (characterized by gene expression of mature hepatoctes and mature hepatocyte functions) by applying the method of the invention.

Using liver progenitor phenotype cells like ALDP cells in the method of the invention most likely facilitates the hepatic differentiation process induced by the transcription factors. The method of the invention has also been applied to NIH 3T3 and mouse embryonic fibroblasts as precursor cells. Although, the efficacy of hepatic differentiation was less than observed for the ALDP cells, however also these cells could be differentiated into hepatocyte like cells. For example, a dramatic morphological change to an epitheliod phenotype was noted. Moreover albumin expression and expression of several other liver genes could be induced in triple transduced fibroblasts. Although most of the parameters tested did not reach the levels of mature hepatocytes, this shows that the methods described herein can also be applied to other cell types (e.g. fibroblasts, mesenchymal cells or virtually any suitable precursor cell type) or cell lines than ALDP cells which are preferably used.

In a preferred embodiment of the method of the invention the obtained cells are free of pathogens.

The term "pathogen" in accordance with the invention refers to a biological agent that causes or contributes to a disease in the host. Examples of pathogens comprise viral pathogens, fungal pathogens, helminthic pathogens, prionic pathogens, protistic pathogens and bacterial pathogens.

In a preferred embodiment of the method of the invention the cells having a mature hepatocyte phenotype are characterized by the expression of albumin, AAT, TAT, TDO, G6P, Cyp3A7, Cyp2A5, PXR, Apo44, ApoC3 and ApoA1.

The expression of the genes albumin, AAT, TAT, TDO, G6P, Cyp3A7, Cyp2A5, PXR, Apo44, ApoC3 and ApoA1 characterize the known gene expression profile of mature hepatocytes (e.g. Li et al. (2010) Differentiation. 2010 Apr 26. [Epub ahead of print], Castro et al. (2005) Journal of Hepatology, 42(6):897-906; or Wu et al. (2002)The FASEB Journal;16:1665-1667).

In a further preferred embodiment of the method of the invention the cells having a mature hepatocyte phenotype are characterized by the capability to store glycogen intracellularly and the capability to generate urea.

The intracellular storage of glycogen and the capability to generate urea inter alia characterize mature hepatocytes (e.g., Michalopoulos et al. (2002), Am J Pathol. 2001 November; 159(5): 1877-1887; Horslen et al (2003), PEDIATRICS, 111;1262-1267, or Baquet et al. (1990), Journal of Biological Chemistry, 265, 955-959.).

In a further preferred embodiment of the method of the invention the cells having a mature hepatocyte phenotype are characterized by comprising cells with dispersed chromatin and/or two or more cell nuclei.

Dispersed chromatin and/or two or more cell nuclei are phenotypic characteristics of mature hepatocytes, well known and described in the art (e.g., Michalopoulos et al. (2002), Am J Pathol. 2001 November; 159(5): 1877-1887).

In a further preferred embodiment of the invention the method of differentiation in step (b) is carried out in the presence of one or more additional differentiation factors selected from the group consisting of histone deacetylase inhibitors.

Although the cells carrying with one or more member(s) of the Foxa subfamily, HNF-4α and C/EBPα exhibit gene expression and protein secretion levels compatible with a mature hepatic phenotype, more complex metabolic functions of hepatocytes such as ureagenesis may still be-optimized. The over-expression of additional transcription factors and three dimensional cultures on scaffolds may further improve the maturation of these cells. In this regard, histone deacetylase inhibitors, which have been shown to improve hepatic differentiation in embryonic and stem cells appear to be suitable for achieving this purpose, because they are known to have role in hepatocytes differentiation (e.g. Yamashita et al . (2002), Cancer Cell Biology 103(5):572-576 or Armeanu et al. (2005),Journal of Hepatology, 42(2):210-217).

According to an embodiment of the invention the cells obtained by the method according to the invention are used for treating or preventing a liver disease.

Accordingly, also described herein is a method of treating or preventing a liver disease comprising administering an pharmaceutically effective amount of cells obtained by the method according to the invention to a subject in need thereof.

Moreover, described herein is a method of treating or preventing a liver disease in a subject in need thereof comprising (a) isolating a cell population comprising precursor cells of mature hepatocytes, wherein said cell population is isolated from said subject; (b) differentiating precursor cells of the cell population into cells having a mature hepatocyte phenotype by introducing into said precursor cells a group of differentiation factors consisting of (i) one or more member(s) of the Foxa subfamily, (ii) HNF-4α and (iii) C/EBPα; and (c) administering an pharmaceutically effective amount of differentiated cells obtained in (c) to said subject.

As shown in the examples of the application, the cells obtained by the method of the invention have the phenotype of mature hepatocytes, however without being identical to mature hepatocytes. Thus, it can be expected that these cells can substitute or partly substitute for mature hepatocytes in vivo, in particular when used for the treatment or inhibition of a liver disease as defined herein.

The term "liver disease" as used herein refers to any pathological condition or injury involving the loss of hepatocytes, or the dysfunction/loss of function of hepatocytes. Thus, the cells obtained by the method of the invention are capable to supplement or partially supplement for mature hepatocytes and/or the function of mature hepatocytes. Liver diseases comprise but are not limited to liver injury, liver failure (i.e. a dysfunction of the liver to perform its normal synthetic and metabolic function as part of normal physiology and comprises acute liver failure and chronic liver failure), hepatitis, cirrhosis, cancer of the liver, glycogen storage disease type II, Gilbert's syndrome, Budd-Chiari syndrome, primary biliary cirrhosis, primary sclerosing cholangitis, Wilson's disease, hepatic encephalopathy, haemochromatosis, urea cycle defects, and non-alcoholic fatty liver disease.

In another embodiment the invention relates to a method for identifying a compound having an pharmacological, cytotoxic, proliferative, transforming or differentiating effect on cells having a mature hepatocyte phenotype obtained by the method described herein, comprising: (a) contacting said cells having a mature hepatocyte phenotype with a test compound; and (e) determining whether the test compound has one or more of said effects on said cells having a mature hepatocyte phenotype.

The cells having mature hepatocyte phenotype obtained by the method described herein are suitable for pharmacological or scientific research and may replace testing of cells directly obtained from liver and/or animal experiments. In this regard compounds may be tested with regard to their effect to cells having the mature hepatocyte phenotype which has been obtained by the method of the invention. Such effects comprise pharmacological, cytotoxic, proliferative, transforming or differentiating effect. Such effects may be for example monitored by measuring cell proliferation, apoptosis, cell morphology, cell migration, gene expression, cellular protein, or metabolic processes.

The figures show:

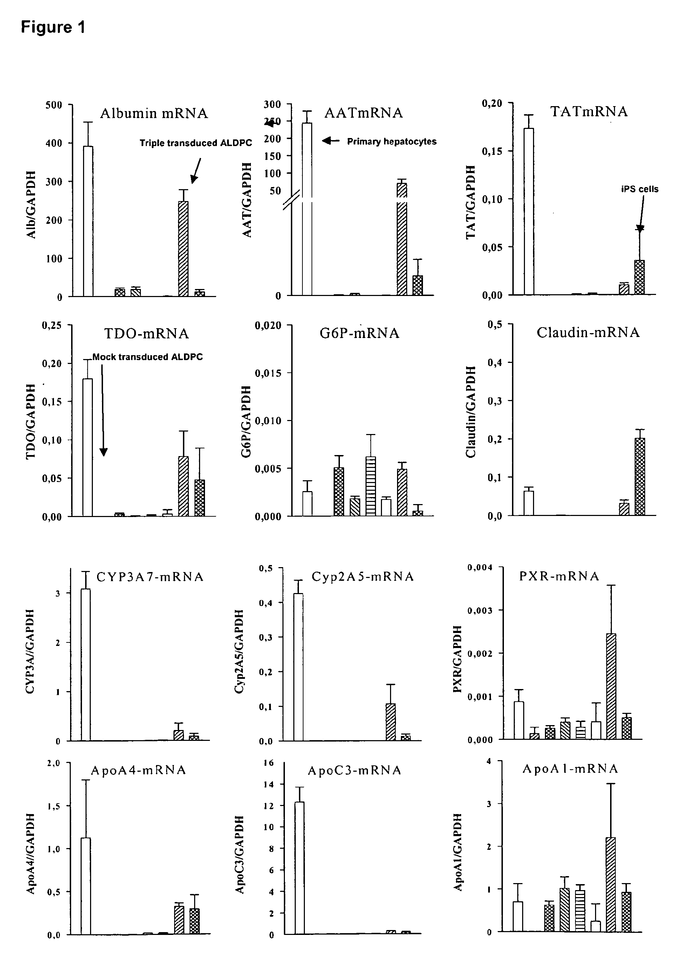

**Figure 1****:** mRNA expression levels relative to GAPDH mRNA expression of the genes albumin, AAT, TAT, TDO, G6P, Claudin1, Cyp3A7, Cyp2A5, PXR, ApoA4, ApoC3, ApoA1 in primary mouse hepatocytes (14h cultured, column 1), ADLPC in basic growth medium condition (column 2), in hepatic differentiation medium LD1, adherent culture(column 3), in differentiation medium LD2, adherent culture (column 4), in differentiation medium LD 1, non-adherent culture (column 5), in differentiation medium LD 2, non-adherent culture (column 6), in LD1 after triple transduction with transcription factors (FoxA2, HNF4, C/EBPα), adherent culture (column 7) and in iPS cells differentiated towards the hepatic lineage (column 8).
**Figure 2****:** mRNA expression levels relative to GAPDH mRNA expression of the transcription factors Foxa2, HNF4 and C7EBPα after lentiviral transduction of the respective cDNAs.

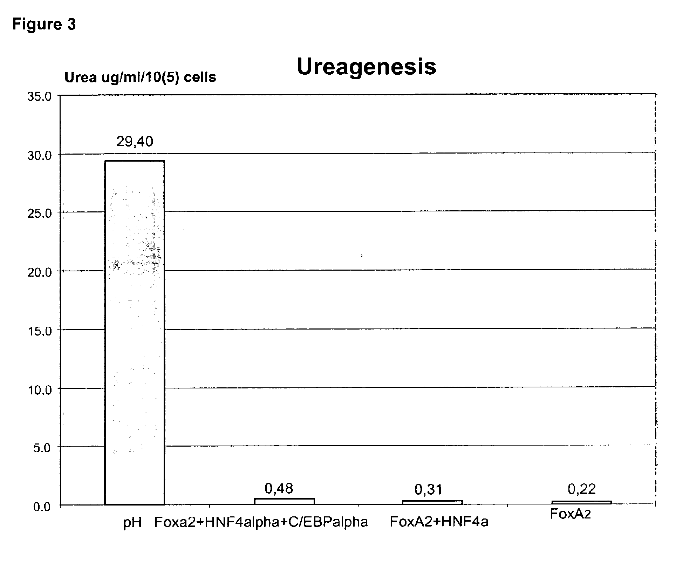

**Figure 3****:** Urea production in ug/ml/10(5) cells in primary hepatocytes (24 hours in culture, ADLPC with three transcription factors (Foxa2, HNF4, C/EBPα) two transcription factors (Foxa2, HNF4) and one transcription factor (FoxA2).

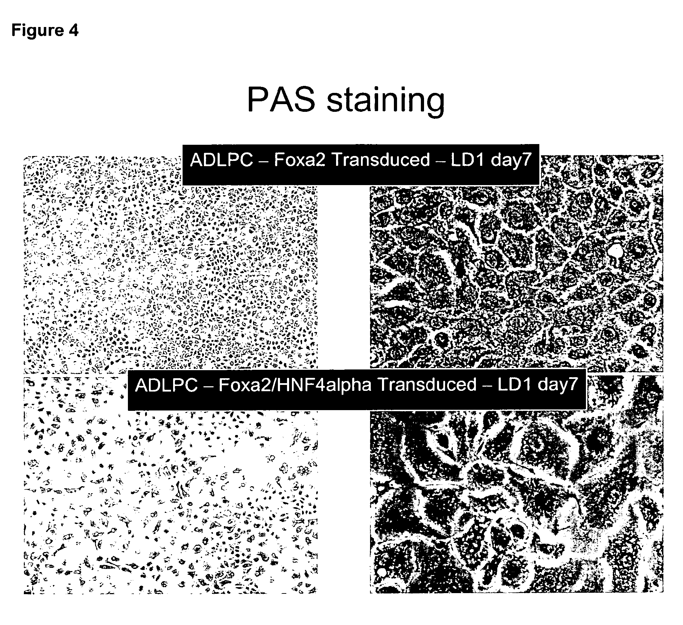

**Figure 4****:** PAS staining in Foxa2 transduced ADLPC cultured in LD1 medium (upper panel) and in Foxa2/HNF4 transduced ADLPC (lower panel). Left microscopic image (x200), right image (x400).

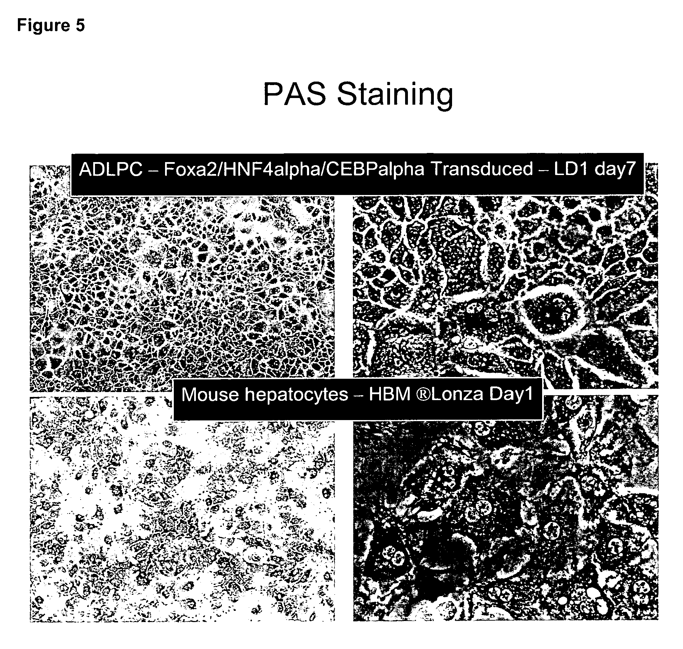

**Figure 5****:** PAS staining in Foxa2/HNF4/C/EBPα transduced ADLPC cultured in LD1 medium (upper panel). PAS staining in cultured primary mouse hepatocytes Left microscopic image (x200), right microscopic image (x400.)
**Figure 6:** (A) Morphological appearance of ADLPC after triple transduction (phase contrast and fluorescence analysis). (B) Mock transduced ADLP.

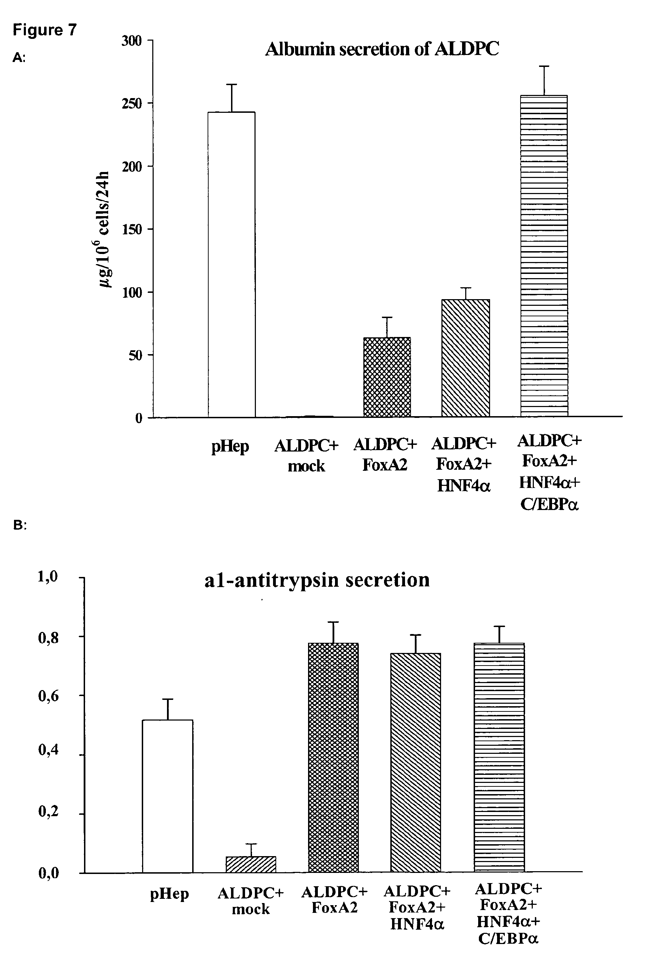

**Figure 7:** (A) Concentration of mouse albumin in the culture supernatant as determined by ELISA (B) Concentration of AAT in the culture supernatant as determined by ELISA.
**Figure 8****:** Lentivirus plasmid with expression cassette containing HNF4 and eGFP cDNA's (upper figure) or eGFP alone (lower figure, control)
**Figure 9****:** Lentivirus plasmid with expression cassette containing C/EBPalpha and diTomato cDNA's (upper figure) or ditomato alone (lower figure, control)
**Figure 10****:** Lentivirus plasmid with expression cassette containing Foxa2 and puromycin cDNA's (upper figure) or puromycin alone (lower figure, control)

The Examples illustrate the invention.

### Example 1 - Experimental procedures

### Lentivirus vectors

Mouse transcription factor cDNA's were derived from a commercial cDNA library (Foxa2, C/EBPα from imaGenes^{®}) and an adult mouse liver RNA sample (HNF4α), and cloned into the BamHI cloning site of a 3^{rd} generation lentiviral expression plasmid containing a SFFV promoter driven IRES-Puro (Foxa2), IRES-dTomato (C/EBPα) or IRES-eGFP (HNF4α) expression cassette, (Supplemental Figure 1). The cDNA's were re-derived from the lentivirus vectors by restriction enzyme digestion and sequenced (SeqLab^{®}, Göttingen) for quality control before virus production.
Lentiviruses expressing the transcription factors or the respective "empty" controls were produced by transient transfection of HEK293T cells according to standard protocols. Virus containing supernatants were concentrated by centrifugation using a Centricon Plus-70 filter device (Millipore^{®}) according to the manufacturers protocol. Virus titers were estimated by transduction of NIH3T3 cells using increments of the enriched culture supernatants.

### Transcription factor reporter assays

Transcription factor reporter assays were performed using the Dual Luciferase reporter assay system according to manufacturers protocol (Promega^{®}) For estimation of the HNF4α and C/EBPα transcriptional activities a luciferase reporter plasmid (pGL3-basic) containing a 530 bp sequence from the UGT1A9 promoter was utilized.
For the assay, 5x10⁴ HEK293T cells were seeded in 12-well plates and transfected with 1,5 ug lipofectamine (Invitrogen^{®}) complexed DNA/well (1:1 ratio of the lentiviral transcription factor plasmid and the UGT1A9 reporter plasmid) at 70% confluency. The pRL-TK vector DNA (Promega ®) expressing Renilla luciferase (19,8 ng/well) was co-transfected as internal control. All experiments were performed in triplicates and empty SFFV-Ires-eGFP and SFFV-Ires-dTomato plasmids were used as negative controls. Firefly/Renilla luminescence ratio was analyzed 36 hours after transfection in all experiments.

### Hepatocyte isolation and culture

Hepatocytes were isolated by a modified 2-step collagenase perfusion. The liver was first perfused with EGTA solution (5.4 mmol/L KCI, 0.44 mmol/L KH2PO4, 140 mmol/L NaCl, 0.34 mmol/L Na2HPO4,0.5 mmol/L EGTA, and 25 mmol/L Tricine, pH 7.2) with a pump rate of 8 ml/min. Subsequently liver digest medium was applied for enzymatic digestion of the tissue at 37°C and was supplemented with Serva Collagenase NB-4 (Serva Electrophoresis GmbH, Heidelberg) at concentrations of 400 - 480 mg/L depending on the lot specific properties. After digestion the tissue was manually disrupted with sterile scissors and scalpels in Williams E Medium (PAN Biotech GmbH, Aidenbach). To separate undigested tissue pieces, the suspended hepatocytes were passed through a 100 µm filter into 50 ml Falcon tubes. The cell suspensions were centrifuged twice at 50 g for 5 minutes at 4°C and the cell pellet was resuspended in Williams E Medium (PAN Biotech GmbH, Aidenbach). An aliquot of the cell preparation was separated for cell count and viability analysis (light microscopy and trypan blue exclusion test).

### Adult mouse liver clonal cell lines

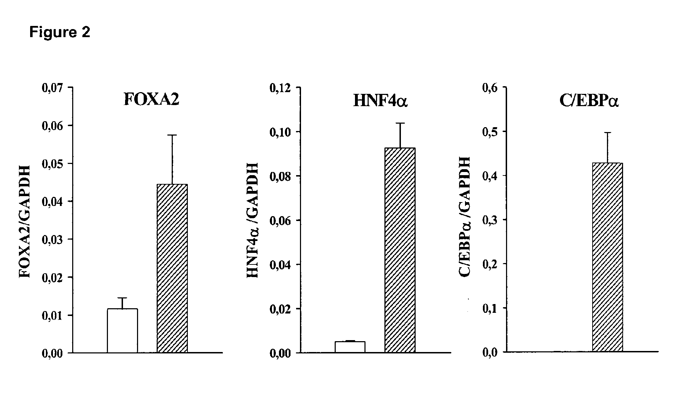

Adult liver derived progenitor cells (ALDPC) were obtained from cultured adult mouse liver cell suspensions by the "plate and wait" technique, according to published protocols (Fougere, Strick Marchand, Deschartrette 2006). The liver cell suspension was plated on a rat tail collagen I layer in hepatocyte basal medium HCM ® plus supplements (Lonza, Switzerland) and 10% fetal calf serum. Twenty four hours later the medium was changed to Williams E medium containing 50ng of epidermal growth factor (EGF) and 30 ng insulin growth factor 2 (IGF-2) and 10 ng/ml insulin. At day seven colonies of small transparent cells with an epitheloid morphology became visible. A confluent monolayer of cells was formed after three weeks of culture. After re-cloning stable and homogeneous cell cultures of ALDPC's were generated. All cell clones derived from mature mouse liver cell suspension expressed CK18 and CK 19 mRNA (see supplement Fig.2). For the experiments the ALDP clone E at passage 40 was used.

### Hepatic differentiation of ALDPC's and TF transduced ALDPC's

Hepatic differentiation was induced in adherent Clone E cells, over a period of 7 days. Cells were seeded at a density of 1.5 X 10⁵ cells/well in rat tail collagen-coated 6 well plates (Roche^{®}) using two different liver differentiation media: HCM medium (Lonza^{®}) supplemented with the standard single quots^{®} of insulin, hydrocortisone, transferin, antibiotics and mouse hepatocyte growth factor (HGF, 20ng/ml) (medium LD1) or HCM medium supplemented with the standard single quots^{®} of insulin, transferin, antibiotics, dexamethasone (40 ng/ml), mouse HGF (20 ng/ml) and oncostatin M (20 ng/ml) (medium LD2). The same experiments were repeated in ultra low adherence culture conditions (Costar^{®}) in order to allow formation of cellular aggregates.
As a first transduction step, a *Foxa2* transgenic ALDPC's cell line was established by transducing passage no. 40 expanded cells with the Foxa2 expressing lentivirus and puromycine selection (2µg/ml). After obtaining a proliferating transgenic cell population, puromycine selection was continued for 3 weeks (1ug/ml puromycine) to assure optimal selection of transduced cells. During this step, cells were grown in Williams E media supplemented with 10% FCS, 50 ng/ml epidermal growth factor (EGF), 30ng/ml insulin-like growth factor II (IGFII) (PeproTech^{®}) and 10 ng/ml insulin (Sigma Aldrich ®).
In a second step 1.5x10⁵ *Foxa2* transgenic ALDPC's were seeded on rat tail collagen-coated 6 well plates (Roche^{®}), cultured in liver differentiation media LD1 and co-transduced with either SFFV-HNF4alpha-IRES-eGFP and SFFV-C/EBPalpha-IRES-dTomato or with SFFV-Ires-eGFP and SFFV-Ires-dTomato control lentiviruses in adherent] culture conditions. For the transduction, a lentivirus titer of 1.8 x 10⁵ was used in 1 ml total transduction volume. Single transductions with SFFV-HNF4alpha-IRES-eGFP or SFFV-C/EBPalpha-IRES-dTomato have also been performed.

### Enzyme linked immunosorbent assay

The amount of albumin secretion was quantified by sandwich enzyme linked immunosorbent assay using a mouse albumin ELISA quantitation kit according to manufacturers protocol (Bethyl Laboratories, Inc, TX, USA).
The mouse alpha-1 antitrypsin (mAAT) ELISA, was performed using cell culture supernatants in 96 well plates (EIA/ RIA 96well, Costar) using a monoclonal antibody against murine AAT (Abcam). After incubation with a secondary HRP labelled antibody (Abcam) the substrate TMB/H₂O₂ was added. The colour development was stopped with 1 N sulfuric acid after 60min followed by photometric analysis at 450nm and 540nm.
RNA extraction and quantitative real time PCR. From 24 hours cultured mouse hepatocytes and ALDPC cells
From 24 hours cultured mouse hepatocytes and ALDPCs total RNA was extracted using the RNEasy Mini kit (Qiagen®) and subjected to reverse transcription using iScript™ cDNA synthesis kit (Biorad). The messenger RNA (mRNA) expression levels were determined by quantitative RT-PCR by a LightCycler II System (Roche Applied Science) and gene specific primers. For standardization of the qRT-PCR analysis, the PCR Fragment of each gene was cloned in the pCR4-Topo vector (Invitrogen) and sequenced. Standard curves for all genes were generated by diluting equal starting concentrations of the plasmids in a pooled sample. The concentrations of the mRNAs were calculated from these standard curves and normalized to GAPDH mRNA expression.

### Analysis of urea production

Ureagenic capacity of differentiated cells was assessed using the QuantiChrom™ Urea Assay kit (BioAssay Systems, CA, USA). Differentiated cells or hepatocyte cultures were incubated for 24 hours with 5mM ammonium chloride and the amount of urea secreted into the culture medium was measured according to the manufacturers protocol. The assay utilizes a chromogenic reagent that forms a coloured complex specifically with urea. The intensity of the colour, measured at 430 nm is directly proportional to the urea concentration in the sample.

### PAS staining

The glycogen storage capacity of the differentiated ALDPC's and control hepatocytes was assessed PAS staining. Briefly, six-well adherent cell cultures were fixed with 95% ethanol for 10 minutes, treated with periodic acid, stained with Shiffs reagent and finally counterstained with Meyer's Hemalaun solution.

### Example 2 - Characterisation of ALDP cells in baseline conditions

ALDP cells were derived from several primary liver cell cultures in medium containing EGF, IGF2 and insulin. A tightly packed monolayer of cells with epitheloid morphology was formed after three to six weeks of culture. Analysis of several ALDP clones showed uniform expression of cytokeratins 7, 8, 18 and 19 mRNA's, E-and N-Cadherin, low levels of liver enriched transcription factor mRNA's including HNF-1α, Foxa2 and HNF4α and no detectable concentrations of C/EBPα mRNA. Only trace amounts of albumin, α1-antitrypsin and α-fetoprotein were secreted into the supernatant. From the twelve mRNA's, which were chosen for differential expression in mouse hepatocytes only the PXR mRNA was detectable at a range close to primary hepatocytes.

### Example 3 - Hepatic differentiation of ALDP cells

The ALDP's, also named BAML or LSC's by others, have been proposed as liver progenitor cells. First tested was the hepatic differentiation capacity in the presence of factors and conditions frequently utilized for unidirectional differentiation to hepatocytes by creating 4 different differentiation settings as described in the methods section: two liver differentiation media (LD1 and LD2) were assessed using adherent or ultra-low adherent culture conditions.
Compared to baseline conditions no significant differences in secreted albumin, AFP and α1-AAT levels were detected after an induction period of 7 days. Glucose-6 phosphatase and Apolipoprotein A1 mRNA's were strongly induced in all four differentiation conditions (p< 0,001 compared to baseline). Alb, AAT, TAT, TDO, ApoA4 mRNA's, which were not detectable in baseline culture conditions, were induced by the various differentiation conditions at low levels compared to primary hepatocytes. No mRNA induction was observed for the Cytochrom p450 genes, Claudin and ApoC3 indicating an only limited and partial hepatic differentiation potential with conventional protocols. The data did not clearly favour one of the four differentiation protocols applied. Subsequent experiments were thus performed in LD1 medium in adherent cell cultures.

### Example 4- Hepatic differentiation of ALDPC's by transcription factors

The concept of forward programming by sequential expression of defined transcription factors was tested in *Foxa2* transgenic ALDP cells by transduction of LV vectors constitutively expressing murine HNF4α-IRES-eGFP and C/EBPα-IRES-dTomato cDNA's. At the end of the differentiation protocol, flow cytometry analysis has indicated 87% eGFP positive, 60% dTomato positive and 34% double positive cells (mean of a set of 3 independent transduction experiments). Cells transduced with each lentivirus vector significantly showed increased mRNA levels of the respective transcription factors (Fig XY). Compared to the parental cells albumin secretion levels increased stepwise in FOXA2 (single), FOXA2/HNF4α (double) and in FOXA2/HNF4α/C/EBPα (triple) transduced ALDPC's. The triple transduced cells secreted albumin at levels not statistically different from the concentrations measured in supernatants of 24h cultured primary hepatocytes. In contrast, AAT protein secretion reached mature hepatocyte levels already in double transduced cells and was not further stimulated in the triple transduced cell population. (In the figure it looks like there is no difference between single, double and triple transduction)
All of the 12 genes representing a wide range of hepatocyte functions were induced in triple transduced cells. The mRNA's of CYP3A11 and ApoC3, although significantly (p<0,001) induced compared to levels of non-transduced ALDP cells, did not reach the concentrations of mature hepatocytes. The quantities of gene expression relative to GAPDH expression exceeded those of mouse iPS derived hepatocyte like cells for 11 of the 12 genes with the exception of TAT mRNA expression levels.

### Example 5 - Glycogene storage

Storage of glycogen is a characteristic feature of mature hepatocytes. Thus primary mouse hepatocytes, ALDP cells and lentivirus transduced ALDP cells were stained with PAS. Non transduced or Foxa2 transgenic ALDP cells showed inconsistent or only very faint PAS positive staining. Double transduced or triple transduced cells, however, presented a much stronger PAS positive staining, comparable adult day1 cultured hepatocytes. Particulary, the double nucleated cells closely resembling adult hepatocytes by morphology, presented also the brightest positive staining.

### Example 6 - Ureagenesis

Ureagenesis was not detectable in non transduced ALDP cells but detectable in transduced cells at the end of differentiation protocol. Triple transduced cells showed the best ureagenic ability, however, a 60 fold lower urea production was noted, compared to day 1 adult hepatocytes.

### References

Takahashi K, Yamanaka S. Induction of pluripotent stem cells from mouse embryonic and adult fibroblast cultures by defined factors. Cell 2006;126:663-76.
Takahashi K, Tanabe K, Ohnuki M, Narita M, Ichisaka T, Tomoda K, Yamanaka S. Induction of pluripotent stem cells from adult human fibroblasts by defined factors. Cell 2007;131:861-72.
Kyrmizi I, Hatzis P, Katrakili N, Tronche F, Gonzalez FJ, Talianidis I. Plasticity and expanding complexity of the hepatic transcription factor network during liver development. Genes Dev. 2006 Aug 15;20(16):2293-305.
Lemaigre FP. Mechanisms of liver development: concepts for understanding liver disorders and design of novel therapies. Gastroenterology. 2009 Jul;137(1):62-79.
Wederell ED, Bilenky M, Cullum R, Thiessen N, Dagpinar M, Delaney A, Varhol R, Zhao Y, Zeng T, Bernier B, Ingham M, Hirst M, Robertson G, Marra MA, Jones S, Hoodless PA. Global analysis of in vivo Foxa2-binding sites in mouse adult liver using massively parallel sequencing. Nucleic Acids Res. 2008 Aug;36(14):4549-64.
Zaret KS, Grompe M. Generation and regeneration of cells of the liver and pancreas. Science. 2008 Dec 5;322(5907):1490-4.
Tan EH, Ma FJ, Gopinadhan S, Sakban RB, Wang ND. C/EBP alpha knock-in hepatocytes exhibit increased albumin secretion and urea production. Cell Tissue Res. 2007 Dec;330(3):427-35.
Nerlov C. The C/EBP family of transcription factors: a paradigm for interaction between gene expression and proliferation control. Trends Cell Biol. 2007 Jul;17(7):318-24.
Tseng HH, Hwang YH, Yeh KT, Chang JG, Chen YL, Yu HS. Reduced expression of C/EBP alpha protein in hepatocellular carcinoma is associated with advanced tumor stage and shortened patient survival. J Cancer Res Clin Oncol. 2009 Feb;135(2):241-7.
Parviz F, Matullo C, Garrison WD, Savatski L, Adamson JW, Ning G, Kaestner KH, et al. Hepatocyte nuclear factor 4 alpha controls the development of a hepatic epithelium and liver morphogenesis. Nat Genet 2003; 34: 292-296.
Li J, Ning G, Duncan SA. Mammalian hepatocyte differentiation requires the transcription factor HNF-4alpha. Genes Dev 2000; 14: 464-474.
Hayhurst GP, Lee YH, Lambert G, Ward JM, Gonzalez FJ. Hepatocyte nuclear factor 4alpha (nuclear receptor 2A1) is essential for maintenance of hepatic gene expression and lipid homeostasis. Mol Cell Biol 2001;21: 1393-1403.
Kang XQ, Zang WJ, Bao LJ, Li DL, Song TS, Xu XL, et al. Fibroblast growth factor-4 and hepatocyte growth factor induce differentiation of human umbilical cord blood-derived mesenchymal stem cells into hepatocytes. World J Gastroenterol. 2005;11:7461-7465.
Ong SY, Dai H, Leong KW. Hepatic differentiation potential of commercially available human mesenchymal stem cells. Tissue Eng. 2006;12:3477-85.
Sgodda M, Aurich H, Kleist S, Aurich I, König S, Dollinger MM, et al. Hepatocyte differentiation of mesenchymal stem cells from rat peritoneal adipose tissue in vitro and in vivo. Exp Cell Res. 2007;313:2875-2886.
Vierbuchen T, Ostermeier A, Pang ZP, Kokubu Y, Südhof TC, Wernig M. Direct conversion of fibroblasts to functional neurons by defined factors. Nature. 2010 Feb 25;463(7284):1035-41.
Wünsch E. & Heidrich H.C. Physiol. Chemie (1963), 333:149-159
Yamazaki S, Miki K, Hasegawa K, Sata M, Takayama T, Makuuchi M. Sera from liver failure patients and a demethylating agent stimulate transdifferentiation of murine bone marrow cells into hepatocytes in coculture with nonparenchymal liver cells. J Hepatol. 2003;39:17-23.
Ishii K, Yoshida Y, Akechi Y, Sakabe T, Nishio R, Ikeda R, Terabayashi K, Matsumi Y, Gonda K, Okamoto H, Takubo K, Tajima F, Tsuchiya H, Hoshikawa Y, Kurimasa A, Umezawa A, Shiota G. Hepatic differentiation of human bone marrow-derived mesenchymal stem cells by tetracycline-regulated hepatocyte nuclear factor 3beta. Hepatology. 2008 Aug;48(2):597-606.
Inoue Y, Inoue J, Lambert G, Yim SH, Gonzalez FJ. Disruption of hepatic C/EBPalpha results in impaired glucose tolerance and age-dependent hepatosteatosis. J Biol Chem. 2004 Oct 22;279(43):44740-8.
Wang X, Huang G, Mei S, Qian J, Ji J, Zhang J. Over-expression of C/EBP-alpha induces apoptosis in cultured rat hepatic stellate cells depending on p53 and peroxisome proliferator-activated receptor-gamma. Biochem Biophys Res Commun. 2009 Mar 6;380(2):286-91.
Ishizaka S, Shiroi A, Kanda S, Yoshikawa M, Tsujinoue H, Kuriyama S, Hasuma T, Nakatani K, Takahashi K. Development of hepatocytes from ES cells after transfection with the HNF-3beta gene. FASEB J. 2002 Sep;16(11):1444-6
Kubo A, Kim YH, Irion S, Kasuda S, Takeuchi M, Ohashi K, Iwano M, Dohi Y, Saito Y, Snodgrass R, Keller G. The homeobox gene Hex regulates hepatocyte differentiation from embryonic stem cell-derived endoderm. Hepatology. 2010 Feb;51(2):633-41.

## Claims

1. A method for producing cells having a mature hepatocyte phenotype, comprising:
(a) providing a cell population comprising precursor cells of mature hepatocytes, wherein said cell population is obtained from a subject or derived from a cell line; and
(b) introducing into said precursor cells a group of differentiation factors and/or the nucleic acid sequences encoding said differentiation factors, said group consisting of
(i) one or more member(s) of the Foxa subfamily,
(ii) HNF-4α and
(iii) C/EBPα
thereby differentiating said precursor cells into cells having a mature hepatocyte phenotype.

2. The method according to claim 1, further comprising:
(c) isolating cells having a mature hepatocyte phenotype from the cell population obtained in step (b).

3. The method of claim 1 or 2, wherein said introducing into said precursor cells is effected by
(i) transforming said precursor cells with one or more nucleic acids molecule(s) comprising the nucleic acid sequences encoding
(i) one or more member(s) of the Foxa subfamily,
(ii) HNF-4α and
(iii) C/EBPα
in an expressible form; and/or
(ii) microinjection, electroporation, lipofection and/or protein transduction of
(i) one or more member(s) of the Foxa subfamily,
(ii) HNF-4α and
(iii) C/EBPα
into said precursor cells.

4. The method of claim 3, wherein the one or more nucleic acids molecule(s) are one or more vector(s).

5. The method of claim 4, wherein the one or more vector(s) are one or more lentiviral vector(s).

6. The method of any one of claims 3 to 5, wherein the nuclei acid sequences encode (i) the one or more member(s) of the Foxa subfamily, (ii) HNF-4α and (iii) C/EBPα in an inducible expressible form.

7. The method of any one of claims 1 to 6, wherein the member of the Foxa subfamily is Foxa2.

8. The method of any one of claims 1 to 7, wherein the precursor cells of mature hepatocytes are embryonic stem cells, adult stem cells, or iPS cells.

9. The method of any one of claims 1 to 7, wherein the precursor cells of mature hepatocytes are
(a) primitive epithelial progenitor cells obtained from liver suspension, or
(b) adult liver derived progenitor cells.

10. The method of any of one of claims 1 to 9, wherein the obtained cells are free of pathogens.

11. The method of any one of claims 1 to 10, wherein the cells having a mature hepatocyte phenotype are **characterized by** the expression of albumin, AAT, TAT, TDO, G6P, Cyp3A7, Cyp2A5, PXR, Apo44, ApoC3 and ApoA1.

12. The method of any one of claims 1 to 11, wherein the cells having a mature hepatocyte phenotype are **characterized by** the capability to store glycogen intracellularly and the capability to generate urea.

13. The method of any one of claims 1 to 12, wherein the cells having a mature hepatocyte phenotype are **characterized by** comprising cells with dispersed chromatin and/or two or more cell nuclei.

14. The method of any one of claims 1 to 13, wherein the differentiation step (b) is carried out in the presence of one or more additional differentiation factors selected from the group consisting of histone deacetylase inhibitors.

15. The cell obtained by the method according to any one of claims 1 to 14 for use in treating or preventing a liver disease.

16. A method for identifying a compound having an pharmacological, cytotoxic, proliferative, transforming or differentiating effect on cells having a mature hepatocyte phenotype obtained by the method according to any one of claims 1 to 14, comprising:
(a) contacting said cells having a mature hepatocyte phenotype with a test compound; and
(b) determining whether the test compound has one or more of said effects on said cells having a mature hepatocyte phenotype.


SEQUENCE LISTING

<110>  Medizinische Hochschule Hannover

<120>  INDUCTION OF A MATURE HEPATOCYTE PHENOTYPE

<130>  S1485 EP

<160>  20

<170>  PatentIn version 3.3

<210>  1
<211>  1419
<212>  DNA
<213>  Homo sapiens

<400>  1
atgttaggaa ctgtgaagat ggaagggcat gaaaccagcg actggaacag ctactacgca      60

gacacgcagg aggcctactc ctccgtcccg gtcagcaaca tgaactcagg cctgggctcc     120

atgaactcca tgaacaccta catgaccatg aacaccatga ctacgagcgg caacatgacc     180

ccggcgtcct tcaacatgtc ctatgccaac ccgggcctag gggccggcct gagtcccggc     240

gcagtagccg gcatgccggg gggctcggcg ggcgccatga acagcatgac tgcggccggc     300

gtgacggcca tgggtacggc gctgagcccg agcggcatgg gcgccatggg tgcgcagcag     360

gcggcctcca tgaatggcct gggcccctac gcggccgcca tgaacccgtg catgagcccc     420

atggcgtacg cgccgtccaa cctgggccgc agccgcgcgg gcggcggcgg cgacgccaag     480

acgttcaagc gcagctaccc gcacgccaag ccgccctact cgtacatctc gctcatcacc     540

atggccatcc agcaggcgcc cagcaagatg ctcacgctga gcgagatcta ccagtggatc     600

atggacctct tcccctatta ccggcagaac cagcagcgct ggcagaactc catccgccac     660

tcgctgtcct tcaatgactg cttcgtcaag gtggcacgct ccccggacaa gccgggcaag     720

ggctcctact ggacgctgca cccggactcc ggcaacatgt tcgagaacgg ctgctacttg     780

cgccgccaga agcgcttcaa gtgcgagaag cagccggggg ccggcggcgg gggcgggagc     840

ggaagcgggg gcagcggcgc caagggcggc cctgagagcc gcaaggaccc ctctggcgcc     900

tctaacccca gcgccgactc gcccctccat cggggtgtgc acgggaagac cggccagcta     960

gagggcgcgc cggcccccgg gcccgccgcc agcccccaga ctctggacca cagtggggcg    1020

acggcgacag ggggcgcctc ggagttgaag actccagcct cctcaactgc gccccccata    1080

agctccgggc ccggggcgct ggcctctgtg cccgcctctc acccggcaca cggcttggca    1140

ccccacgagt cccagctgca cctgaaaggg gacccccact actccttcaa ccacccgttc    1200

tccatcaaca acctcatgtc ctcctcggag cagcagcata agctggactt caaggcatac    1260

gaacaggcac tgcaatactc gccttacggc tctacgttgc ccgccagcct gcctctaggc    1320

agcgcctcgg tgaccaccag gagccccatc gagccctcag ccctggagcc ggcgtactac    1380

caaggtgtgt attccagacc cgtcctaaac acttcctag                           1419

<210>  2

<211>  472
<212>  PRT
<213>  Homo sapiens

<400>  2
   Met Leu Gly Thr Val Lys Met Glu Gly His Glu Thr Ser Asp Trp Asn
   1 5 10 15
   Ser Tyr Tyr Ala Asp Thr Gln Glu Ala Tyr Ser Ser Val Pro Val Ser
   20 25 30
   Asn Met Asn Ser Gly Leu Gly Ser Met Asn Ser Met Asn Thr Tyr Met
   35 40 45
   Thr Met Asn Thr Met Thr Thr Ser Gly Asn Met Thr Pro Ala Ser Phe
   50 55 60
   Asn Met Ser Tyr Ala Asn Pro Gly Leu Gly Ala Gly Leu Ser Pro Gly
   65 70 75 80
   Ala Val Ala Gly Met Pro Gly Gly Ser Ala Gly Ala Met Asn Ser Met
   85 90 95
   Thr Ala Ala Gly Val Thr Ala Met Gly Thr Ala Leu Ser Pro Ser Gly
   100 105 110
   Met Gly Ala Met Gly Ala Gln Gln Ala Ala Ser Met Asn Gly Leu Gly
   115 120 125
   Pro Tyr Ala Ala Ala Met Asn Pro Cys Met Ser Pro Met Ala Tyr Ala
   130 135 140
   Pro Ser Asn Leu Gly Arg Ser Arg Ala Gly Gly Gly Gly Asp Ala Lys
   145 150 155 160
   Thr Phe Lys Arg Ser Tyr Pro His Ala Lys Pro Pro Tyr Ser Tyr Ile
   165 170 175
   Ser Leu Ile Thr Met Ala Ile Gln Gln Ala Pro Ser Lys Met Leu Thr
   180 185 190
   Leu Ser Glu Ile Tyr Gln Trp Ile Met Asp Leu Phe Pro Tyr Tyr Arg
   195 200 205
   Gln Asn Gln Gln Arg Trp Gln Asn Ser Ile Arg His Ser Leu Ser Phe
   210 215 220
   Asn Asp Cys Phe Val Lys Val Ala Arg Ser Pro Asp Lys Pro Gly Lys
   225 230 235 240
   Gly Ser Tyr Trp Thr Leu His Pro Asp Ser Gly Asn Met Phe Glu Asn
   245 250 255
   Gly Cys Tyr Leu Arg Arg Gln Lys Arg Phe Lys Cys Glu Lys Gln Pro
               260                 265                 270
   Gly Ala Gly Gly Gly Gly Gly Ser Gly Ser Gly Gly Ser Gly Ala Lys
           275                 280                 285
   Gly Gly Pro Glu Ser Arg Lys Asp Pro Ser Gly Ala Ser Asn Pro Ser
       290                 295                 300
   Ala Asp Ser Pro Leu His Arg Gly Val His Gly Lys Thr Gly Gln Leu
   305                 310                 315                 320
   Glu Gly Ala Pro Ala Pro Gly Pro Ala Ala Ser Pro Gln Thr Leu Asp
                   325                 330                 335
   His Ser Gly Ala Thr Ala Thr Gly Gly Ala Ser Glu Leu Lys Thr Pro
               340                 345                 350
   Ala Ser Ser Thr Ala Pro Pro Ile Ser Ser Gly Pro Gly Ala Leu Ala
           355                 360                 365
   Ser Val Pro Ala Ser His Pro Ala His Gly Leu Ala Pro His Glu Ser
       370                 375                 380
   Gln Leu His Leu Lys Gly Asp Pro His Tyr Ser Phe Asn His Pro Phe
   385                 390                 395                 400
   Ser Ile Asn Asn Leu Met Ser Ser Ser Glu Gln Gln His Lys Leu Asp
                   405                 410                 415
   Phe Lys Ala Tyr Glu Gln Ala Leu Gln Tyr Ser Pro Tyr Gly Ser Thr
               420                 425                 430
   Leu Pro Ala Ser Leu Pro Leu Gly Ser Ala Ser Val Thr Thr Arg Ser
           435                 440                 445
   Pro Ile Glu Pro Ser Ala Leu Glu Pro Ala Tyr Tyr Gln Gly Val Tyr
       450                 455                 460
   Ser Arg Pro Val Leu Asn Thr Ser
   465                 470

<210>  3
<211>  1392
<212>  DNA
<213>  Homo sapiens

<400>  3
atgcactcgg cttccagtat gctgggagcg gtgaagatgg aagggcacga gccgtccgac      60

tggagcagct actatgcaga gcccgagggc tactcctccg tgagcaacat gaacgccggc     120

ctggggatga acggcatgaa cacgtacatg agcatgtcgg cggccgccat gggcagcggc 180

tcgggcaaca tgagcgcggg ctccatgaac atgtcgtcgt acgtgggcgc tggcatgagc 240

ccgtccctgg cggggatgtc ccccggcgcg ggcgccatgg cgggcatggg cggctcggcc 300

ggggcggccg gcgtggcggg catggggccg cacttgagtc ccagcctgag cccgctcggg 360

gggcaggcgg ccggggccat gggcggcctg gccccctacg ccaacatgaa ctccatgagc 420

cccatgtacg ggcaggcggg cctgagccgc gcccgcgacc ccaagaccta caggcgcagc 480

tacacgcacg caaagccgcc ctactcgtac atctcgctca tcaccatggc catccagcag 540

agccccaaca agatgctgac gctgagcgag atctaccagt ggatcatgga cctcttcccc 600

ttctaccggc agaaccagca gcgctggcag aactccatcc gccactcgct ctccttcaac 660

gactgtttcc tgaaggtgcc ccgctcgccc gacaagcccg gcaagggctc cttctggacc 720

ctgcaccctg actcgggcaa catgttcgag aacggctgct acctgcgccg ccagaagcgc 780

ttcaagtgcg agaagcagct ggcgctgaag gaggccgcag gcgccgccgg cagcggcaag 840

aaggcggccg ccggagccca ggcctcacag gctcaactcg gggaggccgc cgggccggcc 900

tccgagactc cggcgggcac cgagtcgcct cactcgagcg cctccccgtg ccaggagcac 960

aagcgagggg gcctgggaga gctgaagggg acgccggctg cggcgctgag ccccccagag 1020

ccggcgccct ctcccgggca gcagcagcag gccgcggccc acctgctggg cccgccccac 1080

cacccgggcc tgccgcctga ggcccacctg aagccggaac accactacgc cttcaaccac 1140

ccgttctcca tcaacaacct catgtcctcg gagcagcagc accaccacag ccaccaccac 1200

caccaacccc acaaaatgga cctcaaggcc tacgaacagg tgatgcacta ccccggctac 1260

ggttccccca tgcctggcag cttggccatg ggcccggtca cgaacaaaac gggcctggac 1320

gcctcgcccc tggccgcaga tacctcctac taccaggggg tgtactcccg gcccattatg 1380

aactcctctt aa 1392

<210> 4
<211> 463
<212> PRT
<213> Homo sapiens

<400> 4

<210>   5
<211>   1053
<212>   DNA
<213>   Homo sapiens

<400>   5
atgctgggct cagtgaagat ggaggcccat gacctggccg agtggagcta ctacccggag      60

gcgggcgagg tctactcgcc ggtgacccca gtgcccacca tggcccccct caactcctac      120

atgaccctga atcctctaag ctctccctat ccccctgggg ggctccctgc ctccccactg      180

ccctcaggac ccctggcacc cccagcacct gcagcccccc tggggcccac tttcccaggc      240

ctgggtgtca gcggtggcag cagcagctcc gggtacgggg ccccgggtcc tgggctggtg      300

cacgggaagg agatgccgaa ggggtatcgg cggcccctgg cacacgccaa gccaccgtat      360

tcctatatct cactcatcac catggccatc cagcaggcgc cgggcaagat gctgaccttg      420

agtgaaatct accagtggat catggacctc ttcccttact accgggagaa tcagcagcgc      480

tggcagaact ccattcgcca ctcgctgtct ttcaacgact gcttcgtcaa ggtggcgcgt      540

tccccagaca agcctggcaa gggctcctac tgggccctac accccagctc agggaacatg      600

tttgagaatg gctgctacct gcgccgccag aaacgcttca agctggagga gaaggtgaaa      660

aaagggggca gcggggctgc caccaccacc aggaacggga cagggtctgc tgcctcgacc      720

accacccccg cggccacagt cacctccccg ccccagcccc cgcctccagc ccctgagcct      780

gaggcccagg gcggggaaga tgtgggggct ctggactgtg gctcacccgc ttcctccaca      840

ccctatttca ctggcctgga gctcccaggg gagctgaagc tggacgcgcc ctacaacttc      900

aaccaccctt tctccatcaa caacctaatg tcagaacaga caccagcacc tcccaaactg 960
gacgtggggt ttgggggcta cggggctgaa ggtggggagc ctggagtcta ctaccagggc 1020
ctctattccc gctctttgct taatgcatcc tag 1053

<210> 6
<211> 350
<212> PRT
<213> Homo sapiens

<400> 6

Met Leu Gly Ser Val Lys Met Glu Ala His Asp Leu Ala Glu Trp Ser
1               5                   10                  15

Tyr Tyr Pro Glu Ala Gly Glu Val Tyr Ser Pro Val Thr Pro Val Pro
            20                  25                  30

Thr Met Ala Pro Leu Asn Ser Tyr Met Thr Leu Asn Pro Leu Ser Ser
        35                  40                  45

Pro Tyr Pro Pro Gly Gly Leu Pro Ala Ser Pro Leu Pro Ser Gly Pro
    50                  55                  60

Leu Ala Pro Pro Ala Pro Ala Ala Pro Leu Gly Pro Thr Phe Pro Gly
65                  70                  75                  80

Leu Gly Val Ser Gly Gly Ser Ser Ser Ser Gly Tyr Gly Ala Pro Gly
                85                  90                  95

Pro Gly Leu Val His Gly Lys Glu Met Pro Lys Gly Tyr Arg Arg Pro
            100                 105                 110

Leu Ala His Ala Lys Pro Pro Tyr Ser Tyr Ile Ser Leu Ile Thr Met
        115                 120                 125

Ala Ile Gln Gln Ala Pro Gly Lys Met Leu Thr Leu Ser Glu Ile Tyr
    130                 135                 140

Gln Trp Ile Met Asp Leu Phe Pro Tyr Tyr Arg Glu Asn Gln Gln Arg
145                 150                 155                 160

Trp Gln Asn Ser Ile Arg His Ser Leu Ser Phe Asn Asp Cys Phe Val
                165                 170                 175

Lys Val Ala Arg Ser Pro Asp Lys Pro Gly Lys Gly Ser Tyr Trp Ala
            180                 185                 190

Leu His Pro Ser Ser Gly Asn Met Phe Glu Asn Gly Cys Tyr Leu Arg
        195                 200                 205

Arg Gln Lys Arg Phe Lys Leu Glu Glu Lys Val Lys Lys Gly Gly Ser
    210                 215                 220

Gly Ala Ala Thr Thr Thr Arg Asn Gly Thr Gly Ser Ala Ala Ser Thr
225                 230                 235                 240

Thr Thr Pro Ala Ala Thr Val Thr Ser Pro Pro Gln Pro Pro Pro Pro
                245                 250                 255

Ala Pro Glu Pro Glu Ala Gln Gly Gly Glu Asp Val Gly Ala Leu Asp
            260                 265                 270

Cys Gly Ser Pro Ala Ser Ser Thr Pro Tyr Phe Thr Gly Leu Glu Leu
        275                 280                 285

Pro Gly Glu Leu Lys Leu Asp Ala Pro Tyr Asn Phe Asn His Pro Phe
    290                 295                 300

Ser Ile Asn Asn Leu Met Ser Glu Gln Thr Pro Ala Pro Pro Lys Leu
305                 310                 315                 320

Asp Val Gly Phe Gly Gly Tyr Gly Ala Glu Gly Gly Glu Pro Gly Val
                325                 330                 335

Tyr Tyr Gln Gly Leu Tyr Ser Arg Ser Leu Leu Asn Ala Ser
            340                 345                 350

<210>  7
<211>  1077
<212>  DNA
<213>  Homo sapiens

<400>  7
atggagtcgg ccgacttcta cgaggcggag ccgcggcccc cgatgagcag ccacctgcag      60

agccccccgc acgcgcccag cagcgccgcc ttcggctttc cccggggcgc gggccccgcg      120

cagcctcccg ccccacctgc cgccccggag ccgctgggcg gcatctgcga gcacgagacg      180

tccatcgaca tcagcgccta catcgacccg gccgccttca acgacgagtt cctggccgac      240

ctgttccagc acagccggca gcaggagaag gccaaggcgg ccgtgggccc cacgggcggc      300

ggcggcggcg gcgactttga ctacccgggc gcgcccgcgg gccccggcgg cgccgtcatg      360

cccgggggag cgcacgggcc cccgcccggc tacggctgcg cggccgccgg ctacctggac      420

ggcaggctgg agcccctgta cgagcgcgtc ggggcgccgg cgctgcggcc gctggtgatc      480

aagcaggagc cccgcgagga ggatgaagcc aagcagctgg cgctggccgg cctcttccct      540

taccagccgc cgccgccgcc gccgccctcg cacccgcacc cgcacccgcc gcccgcgcac      600

ctggccgccc cgcacctgca gttccagatc gcgcactgcg gccagaccac catgcacctg      660

cagcccggtc accccacgcc gccgcccacg cccgtgccca gcccgcaccc cgcgcccgcg      720

ctcggtgccg ccggcctgcc gggccctggc agcgcgctca aggggctggg cgccgcgcac      780

cccgacctcc gcgcgagtgg cggcagcggc gcgggcaagg ccaagaagtc ggtggacaag      840

aacagcaacg agtaccgggt gcggcgcgag cgcaacaaca tcgcggtgcg caagagccgc     900

gacaaggcca agcagcgcaa cgtggagacg cagcagaagg tgctggagct gaccagtgac     960

aatgaccgcc tgcgcaagcg ggtggaacag ctgagccgcg aactggacac gctgcggggc    1020

atcttccgcc agctgccaga gagctccttg gtcaaggcca tgggcaactg cgcgtga       1077

<210>  8
<211>  358
<212>  PRT
<213>  Homo sapiens

<400>  8

<210>  9
<211>  1425
<212>  DNA
<213>  Homo sapiens

<400>  9
atgcgactct ccaaaaccct cgtcgacatg gacatggccg actacagtgc tgcactggac      60

ccagcctaca ccaccctgga atttgagaat gtgcaggtgt tgacgatggg caatgacacg     120

tccccatcag aaggcaccaa cctcaacgcg cccaacagcc tgggtgtcag cgccctgtgt     180

gccatctgcg gggaccgggc cacgggcaaa cactacggtg cctcgagctg tgacggctgc     240

aagggcttct tccggaggag cgtgcggaag aaccacatgt actcctgcag atttagccgg     300

cagtgcgtgg tggacaaaga caagaggaac cagtgccgct actgcaggct caagaaatgc     360

ttccgggctg gcatgaagaa ggaagccgtc cagaatgagc gggaccggat cagcactcga     420

aggtcaagct atgaggacag cagcctgccc tccatcaatg cgctcctgca ggcggaggtc     480

ctgtcccgac agatcacctc ccccgtctcc gggatcaacg gcgacattcg ggcgaagaag     540

attgccagca tcgcagatgt gtgtgagtcc atgaaggagc agctgctggt tctcgttgag     600

tgggccaagt acatcccagc tttctgcgag ctccccctgg acgaccaggt ggccctgctc     660

agagcccatg ctggcgagca cctgctgctc ggagccacca agagatccat ggtgttcaag 720

gacgtgctgc tcctaggcaa tgactacatt gtccctcggc actgcccgga gctggcggag 780

atgagccggg tgtccatacg catccttgac gagctggtgc tgcccttcca ggagctgcag 840

atcgatgaca atgagtatgc ctacctcaaa gccatcatct tctttgaccc agatgccaag 900

gggctgagcg atccagggaa gatcaagcgg ctgcgttccc aggtgcaggt gagcttggag 960

gactacatca acgaccgcca gtatgactcg cgtggccgct ttggagagct gctgctgctg 1020

ctgcccacct tgcagagcat cacctggcag atgatcgagc agatccagtt catcaagctc 1080

ttcggcatgg ccaagattga caacctgttg caggagatgc tgctgggagg gtcccccagc 1140

gatgcacccc atgcccacca ccccctgcac cctcacctga tgcaggaaca tatgggaacc 1200

aacgtcatcg ttgccaacac aatgcccact cacctcagca acggacagat gtgtgagtgg 1260

ccccgaccca ggggacaggc agccacccct gagaccccac agccctcacc gccaggtggc 1320

tcagggtctg agccctataa gctcctgccg ggagccgtcg ccacaatcgt caagcccctc 1380

tctgccatcc cccagccgac catcaccaag caggaagtta tctag 1425

<210> 10
<211> 474
<212> PRT
<213> Homo sapiens

<400> 10

Met Arg Leu Ser Lys Thr Leu Val Asp Met Asp Met Ala Asp Tyr Ser
1               5                   10                  15

Ala Ala Leu Asp Pro Ala Tyr Thr Thr Leu Glu Phe Glu Asn Val Gln
            20                  25                  30

Val Leu Thr Met Gly Asn Asp Thr Ser Pro Ser Glu Gly Thr Asn Leu
        35                  40                  45

Asn Ala Pro Asn Ser Leu Gly Val Ser Ala Leu Cys Ala Ile Cys Gly
    50                  55                  60

Asp Arg Ala Thr Gly Lys His Tyr Gly Ala Ser Ser Cys Asp Gly Cys
65                  70                  75                  80

Lys Gly Phe Phe Arg Arg Ser Val Arg Lys Asn His Met Tyr Ser Cys
                85                  90                  95

Arg Phe Ser Arg Gln Cys Val Val Asp Lys Asp Lys Arg Asn Gln Cys
            100                 105                 110

Arg Tyr Cys Arg Leu Lys Lys Cys Phe Arg Ala Gly Met Lys Lys Glu
        115                 120                 125

Ala Val Gln Asn Glu Arg Asp Arg Ile Ser Thr Arg Arg Ser Ser Tyr
    130                 135                 140

Glu Asp Ser Ser Leu Pro Ser Ile Asn Ala Leu Leu Gln Ala Glu Val
145                 150                 155                 160

Leu Ser Arg Gln Ile Thr Ser Pro Val Ser Gly Ile Asn Gly Asp Ile
                165                 170                 175

Arg Ala Lys Lys Ile Ala Ser Ile Ala Asp Val Cys Glu Ser Met Lys
            180                 185                 190

Glu Gln Leu Leu Val Leu Val Glu Trp Ala Lys Tyr Ile Pro Ala Phe
        195                 200                 205

Cys Glu Leu Pro Leu Asp Asp Gln Val Ala Leu Leu Arg Ala His Ala
    210                 215                 220

Gly Glu His Leu Leu Leu Gly Ala Thr Lys Arg Ser Met Val Phe Lys
225                 230                 235                 240

Asp Val Leu Leu Leu Gly Asn Asp Tyr Ile Val Pro Arg His Cys Pro
                245                 250                 255

Glu Leu Ala Glu Met Ser Arg Val Ser Ile Arg Ile Leu Asp Glu Leu
            260                 265                 270

Val Leu Pro Phe Gln Glu Leu Gln Ile Asp Asp Asn Glu Tyr Ala Tyr
        275                 280                 285

Leu Lys Ala Ile Ile Phe Phe Asp Pro Asp Ala Lys Gly Leu Ser Asp
    290                 295                 300

Pro Gly Lys Ile Lys Arg Leu Arg Ser Gln Val Gln Val Ser Leu Glu
305                 310                 315                 320

Asp Tyr Ile Asn Asp Arg Gln Tyr Asp Ser Arg Gly Arg Phe Gly Glu
                325                 330                 335

Leu Leu Leu Leu Leu Pro Thr Leu Gln Ser Ile Thr Trp Gln Met Ile
            340                 345                 350

Glu Gln Ile Gln Phe Ile Lys Leu Phe Gly Met Ala Lys Ile Asp Asn
        355                 360                 365

Leu Leu Gln Glu Met Leu Leu Gly Gly Ser Pro Ser Asp Ala Pro His
    370                 375                 380

Ala His His Pro Leu His Pro His Leu Met Gln Glu His Met Gly Thr
385                 390                 395                 400

Asn Val Ile Val Ala Asn Thr Met Pro Thr His Leu Ser Asn Gly Gln
                405                 410                 415

Met Cys Glu Trp Pro Arg Pro Arg Gly Gln Ala Ala Thr Pro Glu Thr
            420                 425                 430

Pro Gln Pro Ser Pro Pro Gly Gly Ser Gly Ser Glu Pro Tyr Lys Leu
        435                 440                 445

Leu Pro Gly Ala Val Ala Thr Ile Val Lys Pro Leu Ser Ala Ile Pro
    450                 455                 460

Gln Pro Thr Ile Thr Lys Gln Glu Val Ile
465                 470

<210>  11
<211>  1407
<212>  DNA
<213>  Mus musculus

<400>  11
atgttaggga ctgtgaagat ggaagggcat gagagcaacg actggaacag ctactacgcg      60

gacacgcagg aggcctactc ctctgtccct gtcagcaaca tgaactccgg cctgggctct     120

atgaactcca tgaacaccta catgaccatg aacaccatga ccacgagcgg caacatgacc     180

ccggcttcct tcaacatgtc ctacgccaac acgggcttag gggccggcct gagtcccggt     240

gctgtggctg gcatgccagg gggctctgca ggcgccatga acagcatgac tgcggcgggc     300

gtcacggcca tgggtacggc gctgagcccg ggaggcatgg gctccatggg cgcgcagccc     360

gccacctcca tgaacggcct gggtccctac gccgccgcca tgaacccgtg catgagtccc     420

atggcgtacg cgccgtccaa cctgggccgc agccgcgcgg ggggcggcgg cgacgccaag     480

acattcaagc gcagctaccc tcacgccaag ccgccttact cctacatctc gctcatcacg     540

atggccatcc agcaggcgcc cagcaagatg ctcacgctga gcgagatcta ccagtggatc     600

atggacctct tcccctatta ccgccagaac cagcagcgct ggcagaactc catccgccac     660

tcgctgtcct tcaacgattg tttcgtcaag gtggcacgat ccccggacaa gccaggcaag     720

ggctcctact ggacgctgca cccggactcc ggcaacatgt tcgagaacgg ctgctacttg     780

cgccgccaaa agcgcttcaa gtgtgagaag cagccggggg ccggaggtgg gagtgggggc     840

ggcggctcca aagggggccc agaaagtcgc aaggacccct caggcccggg gaaccccagc     900

gccgagtcac cccttcaccg gggtgtgcac ggaaaggcta gccagctaga gggcgcgccg     960

gccccagggc ccgccgccag cccccagact ctggaccaca gcggggccac ggcgacaggg    1020

ggcgcttcgg agttgaagtc tccagcgtct tcatctgcgc cccccataag ctccgggcca    1080

ggggcgctag catctgtacc cccctctcac ccggctcacg gcctggcacc ccacgaatct    1140

cagctgcatc tgaaagggga tccccactac tcctttaatc accccttctc catcaacaac    1200

ctcatgtcct cctccgagca acagcacaag ctggacttca aggcatacga gcaggcgctg    1260

cagtactctc cttatggcgc taccttgccc gccagtctgc cccttggcag cgcctcagtg    1320

gccacgagga gccccatcga gccctcagcc ctggagccag cctactacca aggtgtgtat    1380

tccagacccg tgctaaatac ttcctag                                        1407

<210>  12
<211>  468
<212>  PRT
<213>  Mus musculus

<400>  12

Met Leu Gly Thr Val Lys Met Glu Gly His Glu Ser Asn Asp Trp Asn
1               5                   10                  15

Ser Tyr Tyr Ala Asp Thr Gln Glu Ala Tyr Ser Ser Val Pro Val Ser
            20                  25                  30

Asn Met Asn Ser Gly Leu Gly Ser Met Asn Ser Met Asn Thr Tyr Met
        35                  40                  45

Thr Met Asn Thr Met Thr Thr Ser Gly Asn Met Thr Pro Ala Ser Phe
    50                  55                  60

Asn Met Ser Tyr Ala Asn Thr Gly Leu Gly Ala Gly Leu Ser Pro Gly
65                  70                  75                  80

Ala Val Ala Gly Met Pro Gly Ala Ser Ala Gly Ala Met Asn Ser Met
                85                  90                  95

Thr Ala Ala Gly Val Thr Ala Met Gly Thr Ala Leu Ser Pro Gly Gly
            100                 105                 110

Met Gly Ser Met Gly Ala Gln Pro Ala Thr Ser Met Asn Gly Leu Gly
        115                 120                 125

Pro Tyr Ala Ala Ala Met Asn Pro Cys Met Ser Pro Met Ala Tyr Ala
    130                 135                 140

Pro Ser Asn Leu Gly Arg Ser Arg Ala Gly Gly Gly Gly Asp Ala Lys
145                 150                 155                 160

Thr Phe Lys Arg Ser Tyr Pro His Ala Lys Pro Pro Tyr Ser Tyr Ile
                165                 170                 175

Ser Leu Ile Thr Met Ala Ile Gln Gln Ala Pro Ser Lys Met Leu Thr
            180                 185                 190

Leu Ser Glu Ile Tyr Gln Trp Ile Met Asp Leu Phe Pro Tyr Tyr Arg
        195                 200                 205

Gln Asn Gln Gln Arg Trp Gln Asn Ser Ile Arg His Ser Leu Ser Phe
    210                 215                 220

Asn Asp Cys Phe Val Lys Val Ala Arg Ser Pro Asp Lys Pro Gly Lys
225                 230                 235                 240

Gly Ser Tyr Trp Thr Leu His Pro Asp Ser Gly Asn Met Phe Glu Asn
                245                 250                 255

Gly Cys Tyr Leu Arg Arg Gln Lys Arg Phe Lys Cys Glu Lys Gln Pro
            260                 265                 270

Gly Ala Gly Gly Gly Ser Gly Gly Gly Gly Ser Lys Gly Gly Pro Glu
        275                 280                 285

Ser Arg Lys Asp Pro Ser Gly Pro Gly Asn Pro Ser Ala Glu Ser Pro
    290                 295                 300

Leu His Arg Gly Val His Gly Lys Ala Ser Gln Leu Glu Gly Ala Pro
305                 310                 315                 320

Ala Pro Gly Pro Ala Ala Ser Pro Gln Thr Leu Asp His Ser Gly Ala
                325                 330                 335

Thr Ala Thr Gly Gly Ala Ser Glu Leu Lys Ser Pro Ala Ser Ser Ser
            340                 345                 350

Ala Pro Pro Ile Ser Ser Gly Pro Gly Ala Leu Ala Ser Val Pro Pro
        355                 360                 365

Ser His Pro Ala His Gly Leu Ala Pro His Glu Ser Gln Leu His Leu
    370                 375                 380

Lys Gly Asp Pro His Tyr Ser Phe Asn His Pro Phe Ser Ile Asn Asn
385                 390                 395                 400

Leu Met Ser Ser Ser Glu Gln Gln His Lys Leu Asp Phe Lys Ala Tyr
                405                 410                 415

Glu Gln Ala Leu Gln Tyr Ser Pro Tyr Gly Ala Thr Leu Pro Ala Ser
            420                 425                 430

Leu Pro Leu Gly Ser Ala Ser Val Ala Thr Arg Ser Pro Ile Glu Pro
        435                 440                 445

Ser Ala Leu Glu Pro Ala Tyr Tyr Gln Gly Val Tyr Ser Arg Pro Val
    450                 455                 460

Leu Asn Thr Ser
465

<210>  13
<211>  1380
<212>  DNA
<213>  Mus musculus

<400>  13
atgctgggag ccgtgaagat ggaagggcac gagccatccg actggagcag ctactacgcg      60

gagcccgagg gctactcttc cgtgagcaac atgaacgccg gcctggggat gaatggcatg     120

aacacataca tgagcatgtc cgcggctgcc atgggcggcg gttccggcaa catgagcgcg     180

ggctccatga acatgtcatc ctatgtgggc gctggaatga gcccgtcgct agctggcatg     240

tccccgggcg ccggcgccat ggcgggcatg agcggctcag ccggggcggc cggcgtggcg     300

ggcatgggac ctcacctgag tccgagtctg agcccgctcg ggggacaggc ggccggggcc     360

atgggtggcc ttgcccccta cgccaacatg aactcgatga gccccatgta cgggcaggcc     420

ggcctgagcc gcgctcggga ccccaagaca taccgacgca gctacacaca cgccaaacct     480

ccctactcgt acatctcgct catcaccatg gccatccagc agagccccaa caagatgctg     540

acgctgagcg agatctatca gtggatcatg gacctcttcc ctttctaccg gcagaaccag     600

cagcgctggc agaactccat ccgccactct ctctccttca acgactgctt tctcaaggtg     660

ccccgctcgc cagacaagcc tggcaagggc tccttctgga ccctgcaccc agactcgggc     720

aacatgttcg agaacggctg ctacctgcgc cgccagaagc gcttcaagtg tgagaagcaa     780

ctggcactga aggaagccgc gggtgcggcc agtagcggag gcaagaagac cgctcctggg     840

tcccaggcct ctcaggctca gctcggggag gccgcgggct cggcctccga gactccggcg     900

ggcaccgagt ccccccattc cagcgcttct ccgtgtcagg agcacaagcg aggtggccta     960

agcgagctaa agggagcacc tgcctctgcg ctgagtcctc ccgagccggc gccctcgcct    1020

gggcagcagc agcaggctgc agcccacctg ctgggcccac ctcaccaccc aggcctgcca    1080

ccagaggccc acctgaagcc cgagcaccat tacgccttca accacccctt ctctatcaac    1140

aacctcatgt cgtccgagca gcaacatcac cacagccacc accaccatca gccccacaaa    1200

atggacctca aggcctacga acaggtcatg cactacccag ggggctatgg ttcccccatg    1260

ccaggcagct tggccatggg cccagtcacg aacaaagcgg gcctggatgc ctcgcccctg    1320

gctgcagaca cttcctacta ccaaggagtg tactccaggc ctattatgaa ctcatcctaa    1380

<210>  14
<211>  459
<212>  PRT
<213>  Mus musculus

<400>  14

Met Leu Gly Ala Val Lys Met Glu Gly His Glu Pro Ser Asp Trp Ser
1               5                   10                  15

Ser Tyr Tyr Ala Glu Pro Glu Gly Tyr Ser Ser Val Ser Asn Met Asn
            20                  25                  30

Ala Gly Leu Gly Met Asn Gly Met Asn Thr Tyr Met Ser Met Ser Ala
        35                  40                  45

Ala Ala Met Gly Gly Gly Ser Gly Asn Met Ser Ala Gly Ser Met Asn
    50                  55                  60

Met Ser Ser Tyr Val Gly Ala Gly Met Ser Pro Ser Leu Ala Gly Met
65                  70                  75                  80

Ser Pro Gly Ala Gly Ala Met Ala Gly Met Ser Gly Ser Ala Gly Ala
                85                  90                  95

Ala Gly Val Ala Gly Met Gly Pro His Leu Ser Pro Ser Leu Ser Pro
            100                 105                 110

Leu Gly Gly Gln Ala Ala Gly Ala Met Gly Gly Leu Ala Pro Tyr Ala
        115                 120                 125

Asn Met Asn Ser Met Ser Pro Met Tyr Gly Gln Ala Gly Leu Ser Arg
    130                 135                 140

Ala Arg Asp Pro Lys Thr Tyr Arg Arg Ser Tyr Thr His Ala Lys Pro
145                 150                 155                 160

Pro Tyr Ser Tyr Ile Ser Leu Ile Thr Met Ala Ile Gln Gln Ser Pro
                165                 170                 175

Asn Lys Met Leu Thr Leu Ser Glu Ile Tyr Gln Trp Ile Met Asp Leu
            180                 185                 190

Phe Pro Phe Tyr Arg Gln Asn Gln Gln Arg Trp Gln Asn Ser Ile Arg
        195                 200                 205

His Ser Leu Ser Phe Asn Asp Cys Phe Leu Lys Val Pro Arg Ser Pro
    210                 215                 220

Asp Lys Pro Gly Lys Gly Ser Phe Trp Thr Leu His Pro Asp Ser Gly
225                 230                 235                 240

Asn Met Phe Glu Asn Gly Cys Tyr Leu Arg Arg Gln Lys Arg Phe Lys
                245                 250                 255

Cys Glu Lys Gln Leu Ala Leu Lys Glu Ala Ala Gly Ala Ala Ser Ser
            260                 265                 270

Gly Gly Lys Lys Thr Ala Pro Gly Ser Gln Ala Ser Gln Ala Gln Leu
        275                 280                 285

Gly Glu Ala Ala Gly Ser Ala Ser Glu Thr Pro Ala Gly Thr Glu Ser
    290                 295                 300

Pro His Ser Ser Ala Ser Pro Cys Gln Glu His Lys Arg Gly Gly Leu
305                 310                 315                 320

Ser Glu Leu Lys Gly Ala Pro Ala Ser Ala Leu Ser Pro Pro Glu Pro
                325                 330                 335

Ala Pro Ser Pro Gly Gln Gln Gln Gln Ala Ala Ala His Leu Leu Gly
            340                 345                 350

Pro Pro His His Pro Gly Leu Pro Pro Glu Ala His Leu Lys Pro Glu
        355                 360                 365

His His Tyr Ala Phe Asn His Pro Phe Ser Ile Asn Asn Leu Met Ser
    370                 375                 380

Ser Glu Gln Gln His His His Ser His His His His Gln Pro His Lys
385                 390                 395                 400

Met Asp Leu Lys Ala Tyr Glu Gln Val Met His Tyr Pro Gly Gly Tyr
                405                 410                 415

Gly Ser Pro Met Pro Gly Ser Leu Ala Met Gly Pro Val Thr Asn Lys
            420                 425                 430

Ala Gly Leu Asp Ala Ser Pro Leu Ala Ala Asp Thr Ser Tyr Tyr Gln
        435                 440                 445

Gly Val Tyr Ser Arg Pro Ile Met Asn Ser Ser
    450                 455

<210>  15
<211>  1062
<212>  DNA
<213>  Mus musculus

<400>  15
atgctgggct cagtgaagat ggaggctcat gacctggccg agtggagcta ctacccggag      60

gcgggcgagg tgtattctcc agtgaatcct gtgcccacca tggcccctct caactcctac     120

atgaccttga acccactcag ctctccctac cctcccggag ggcttcaggc ctccccactg     180

cctacaggac ccctggcacc cccagccccc actgcgccct tggggcccac cttcccaagc     240

ttgggcactg gtggcagcac cggaggcagt gcttccgggt atgtagcccc agggcccggg     300

cttgtacatg gaaaagagat ggcaaagggg taccggcggc cactggccca cgccaaacca     360

ccatattcct acatctctct cataaccatg gctattcagc aggctccagg caagatgctg     420

accctgagtg aaatctacca atggatcatg gacctcttcc cgtactaccg ggagaaccag     480

caacgttggc agaactccat ccggcattcg ctgtccttca atgactgctt cgtcaaggtg     540

gcacgctccc cagacaagcc aggcaaaggc tcctactggg ccttgcatcc cagctctggg     600

aacatgtttg agaacggctg ctatctccgc cggcagaagc gcttcaagct ggaggagaag     660

gcaaagaaag gaaacagcgc cacatcggcc agcaggaatg gtactgcggg gtcagccacc     720

tctgccacca ctacagctgc cactgcagtc acctccccgg ctcagcccca gcctacgcca     780

tctgagcccg aggcccagag tggggatgat gtggggggtc tggactgcgc ctcacctcct     840

tcgtccacac cttatttcag cggcctggag ctcccggggg aactaaagtt ggatgcgccc     900

tataacttca accacccttt ctctatcaac aacctgatgt cagaacagac atcgacacct     960

tccaaactgg atgtggggtt tgggggctac ggggctgaga gtggggagcc tggagtctac    1020

taccagagcc tctattcccg ctctctgctt aatgcatcct ag                       1062

<210>  16
<211>  353
<212>  PRT
<213>  Mus musculus

<400>  16

Met Leu Gly Ser Val Lys Met Glu Ala His Asp Leu Ala Glu Trp Ser
1               5                   10                  15

Tyr Tyr Pro Glu Ala Gly Glu Val Tyr Ser Pro Val Asn Pro Val Pro
            20                  25                  30

Thr Met Ala Pro Leu Asn Ser Tyr Met Thr Leu Asn Pro Leu Ser Ser
        35                  40                  45

Pro Tyr Pro Pro Gly Gly Leu Gln Ala Ser Pro Leu Pro Thr Gly Pro
    50                  55                  60

Leu Ala Pro Pro Ala Pro Thr Ala Pro Leu Gly Pro Thr Phe Pro Ser
65                  70                  75                  80

Leu Gly Thr Gly Gly Ser Thr Gly Gly Ser Ala Ser Gly Tyr Val Ala
                85                  90                  95

Pro Gly Pro Gly Leu Val His Gly Lys Glu Met Ala Lys Gly Tyr Arg
            100                 105                 110

Arg Pro Leu Ala His Ala Lys Pro Pro Tyr Ser Tyr Ile Ser Leu Ile
        115                 120                 125

Thr Met Ala Ile Gln Gln Ala Pro Gly Lys Met Leu Thr Leu Ser Glu
    130                 135                 140

Ile Tyr Gln Trp Ile Met Asp Leu Phe Pro Tyr Tyr Arg Glu Asn Gln
145                 150                 155                 160

Gln Arg Trp Gln Asn Ser Ile Arg His Ser Leu Ser Phe Asn Asp Cys
                165                 170                 175

Phe Val Lys Val Ala Arg Ser Pro Asp Lys Pro Gly Lys Gly Ser Tyr
            180                 185                 190

Trp Ala Leu His Pro Ser Ser Gly Asn Met Phe Glu Asn Gly Cys Tyr
        195                 200                 205

Leu Arg Arg Gln Lys Arg Phe Lys Leu Glu Glu Lys Ala Lys Lys Gly
    210                 215                 220

Asn Ser Ala Thr Ser Ala Ser Arg Asn Gly Thr Ala Gly Ser Ala Thr
225                 230                 235                 240

Ser Ala Thr Thr Thr Ala Ala Thr Ala Val Thr Ser Pro Ala Gln Pro
                245                 250                 255

Gln Pro Thr Pro Ser Glu Pro Glu Ala Gln Ser Gly Asp Asp Val Gly
            260                 265                 270

Gly Leu Asp Cys Ala Ser Pro Pro Ser Ser Thr Pro Tyr Phe Ser Gly
        275                 280                 285

Leu Glu Leu Pro Gly Glu Leu Lys Leu Asp Ala Pro Tyr Asn Phe Asn
    290                 295                 300

His Pro Phe Ser Ile Asn Asn Leu Met Ser Glu Gln Thr Ser Thr Pro
305                 310                 315                 320

Ser Lys Leu Asp Val Gly Phe Gly Gly Tyr Gly Ala Glu Ser Gly Glu
                325                 330                 335

Pro Gly Val Tyr Tyr Gln Ser Leu Tyr Ser Arg Ser Leu Leu Asn Ala
            340                 345                 350

Ser

<210>   17
<211>   1425
<212>   DNA
<213>   Mus musculus

<400>   17
atgcgactct ctaaaaccct tgccggcatg gatatggccg actacagcgc tgccctggac      60

ccagcctaca ccaccctgga gtttgaaaat gtgcaggtgt tgaccatggg caatgacacg     120

tccccatctg aaggtgccaa cctcaattca tccaacagcc tgggcgtcag tgccctgtgc     180

gccatctgtg gcgaccgggc caccggcaaa cactacggag cctcgagctg tgacggctgc     240

aaggggttct tcaggaggag cgtgaggaag aaccacatgt actcctgcag gtttagccga     300

caatgtgtgg tagacaaaga taagaggaac cagtgtcgtt actgcaggct taagaagtgc     360

ttccgggctg gcatgaagaa ggaagctgtc caaaatgagc gggaccggat cagcacgcgg     420

aggtcaagct acgaggacag cagcctgccc tccatcaacg cgctcctgca ggcagaggtt     480

ctgtcccagc agatcacctc tcccatctct gggatcaatg gcgacattcg ggcaaagaag     540

attgccaaca tcacagacgt gtgtgagtct atgaaggagc agctgctggt cctggtcgag     600

tgggccaagt acatcccggc cttctgcgaa ctccttctgg atgaccaggt ggcgctgctc 660
agggcccacg ccggtgagca tctgctgctt ggagccacca agaggtccat ggtgtttaag 720
gacgtgctgc tcctaggcaa tgactacatc gtccctcggc actgtccaga gctagcggag 780
atgagccgtg tgtccatccg catcctcgat gagctggtcc tgcccttcca agagctgcag 840
attgatgaca atgaatatgc ctgcctcaaa gccatcatct tctttgatcc agatgccaag 900
gggctgagtg acccgggcaa gatcaagcgg ctgcggtcac aggtgcaagt gagcctggag 960
gattacatca acgaccggca gtacgactct cggggccgct ttggagagct gctgctgctg 1020
ttgcccacgc tgcagagcat cacctggcag atgatcgaac agatccagtt catcaagctc 1080
ttcggcatgg ccaagattga caacctgctg caggagatgc ttctcggagg gtctgccagt 1140
gatgcacccc acacccacca ccccctgcac cctcacctga tgcaagaaca catgggcacc 1200
aatgtcattg ttgctaacac gatgccctct cacctcagca atggacagat gtgtgagtgg 1260
ccccgaccca gggggcaggc agccactccc gagactccac agccatcacc accaagtggc 1320
tcgggatctg aatcctacaa gctcctgcca ggagccatca ccaccatcgt caagcctccc 1380
tctgccattc cccagccaac gatcaccaag caagaagcca tctag 1425

<210> 18
<211> 474
<212> PRT
<213> Mus musculus

<400> 18

Met Arg Leu Ser Lys Thr Leu Ala Gly Met Asp Met Ala Asp Tyr Ser
1               5                   10                  15

Ala Ala Leu Asp Pro Ala Tyr Thr Thr Leu Glu Phe Glu Asn Val Gln
            20                  25                  30

Val Leu Thr Met Gly Asn Asp Thr Ser Pro Ser Glu Gly Ala Asn Leu
        35                  40                  45

Asn Ser Ser Asn Ser Leu Gly Val Ser Ala Leu Cys Ala Ile Cys Gly
    50                  55                  60

Asp Arg Ala Thr Gly Lys His Tyr Gly Ala Ser Ser Cys Asp Gly Cys
65                  70                  75                  80

Lys Gly Phe Phe Arg Arg Ser Val Arg Lys Asn His Met Tyr Ser Cys
                85                  90                  95

Arg Phe Ser Arg Gln Cys Val Val Asp Lys Asp Lys Arg Asn Gln Cys
            100                 105                 110

Arg Tyr Cys Arg Leu Lys Lys Cys Phe Arg Ala Gly Met Lys Lys Glu
        115                 120                 125

Ala Val Gln Asn Glu Arg Asp Arg Ile Ser Thr Arg Arg Ser Ser Tyr
    130                 135                 140

Glu Asp Ser Ser Leu Pro Ser Ile Asn Ala Leu Leu Gln Ala Glu Val
145                 150                 155                 160

Leu Ser Gln Gln Ile Thr Ser Pro Ile Ser Gly Ile Asn Gly Asp Ile
                165                 170                 175

Arg Ala Lys Lys Ile Ala Asn Ile Thr Asp Val Cys Glu Ser Met Lys
            180                 185                 190

Glu Gln Leu Leu Val Leu Val Glu Trp Ala Lys Tyr Ile Pro Ala Phe
        195                 200                 205

Cys Glu Leu Leu Leu Asp Asp Gln Val Ala Leu Leu Arg Ala His Ala
    210                 215                 220

Gly Glu His Leu Leu Leu Gly Ala Thr Lys Arg Ser Met Val Phe Lys
225                 230                 235                 240

Asp Val Leu Leu Leu Gly Asn Asp Tyr Ile Val Pro Arg His Cys Pro
                245                 250                 255

Glu Leu Ala Glu Met Ser Arg Val Ser Ile Arg Ile Leu Asp Glu Leu
            260                 265                 270

Val Leu Pro Phe Gln Glu Leu Gln Ile Asp Asp Asn Glu Tyr Ala Cys
        275                 280                 285

Leu Lys Ala Ile Ile Phe Phe Asp Pro Asp Ala Lys Gly Leu Ser Asp
    290                 295                 300

Pro Gly Lys Ile Lys Arg Leu Arg Ser Gln Val Gln Val Ser Leu Glu
305                 310                 315                 320

Asp Tyr Ile Asn Asp Arg Gln Tyr Asp Ser Arg Gly Arg Phe Gly Glu
                325                 330                 335

Leu Leu Leu Leu Leu Pro Thr Leu Gln Ser Ile Thr Trp Gln Met Ile
            340                 345                 350

Glu Gln Ile Gln Phe Ile Lys Leu Phe Gly Met Ala Lys Ile Asp Asn
        355                 360                 365

Leu Leu Gln Glu Met Leu Leu Gly Gly Ser Ala Ser Asp Ala Pro His
    370                 375                 380

Thr His His Pro Leu His Pro His Leu Met Gln Glu His Met Gly Thr
385                 390                 395                 400

Asn Val Ile Val Ala Asn Thr Met Pro Ser His Leu Ser Asn Gly Gln
                405                 410                 415

Met Cys Glu Trp Pro Arg Pro Arg Gly Gln Ala Ala Thr Pro Glu Thr
            420                 425                 430

Pro Gln Pro Ser Pro Pro Ser Gly Ser Gly Ser Glu Ser Tyr Lys Leu
        435                 440                 445

Leu Pro Gly Ala Ile Thr Thr Ile Val Lys Pro Pro Ser Ala Ile Pro
    450                 455                 460

Gln Pro Thr Ile Thr Lys Gln Glu Ala Ile
465                 470

<210>  19
<211>  1080
<212>  DNA
<213>  Mus musculus

<400>  19
atggagtcgg ccgacttcta cgaggtggag ccgcggcccc cgatgagcag tcacctccag      60

agccccccgc acgcgcccag caacgccgcc tttggctttc cccggggcgc gggccccgcg      120

ccgcccccag ccccacctgc cgccccggag ccgctgggcg gcatctgcga gcacgagacg      180

tctatagaca tcagcgccta catcgacccg gccgccttca acgacgagtt cctggccgac      240

ctcttccagc acagccgaca gcaggagaag gccaaggcgg cggcgggccc cgcgggtggc      300

ggcggtgact ttgactaccc gggagccccg gcgggccccg gcggcgcggt catgtccgcg      360

ggggcgcacg ggccccctcc cggctacggc tgtgcggcgg ccggctacct ggacggcagg      420

ctggagcccc tgtacgagcg cgtcggggcg cccgcgctac ggccgctggt gatcaaacaa      480

gagccccgcg aggaggacga ggcgaagcag ctggcgctgg ccggcctctt cccctaccag      540

ccaccgccgc caccgccacc gccgcacccg cacgcgtctc ccgcgcacct ggccgccccc      600

cacttgcagt tccagatcgc gcactgcggc cagaccacca tgcacctgca gcctggccac      660

cccacaccgc cgcccacgcc cgtgcccagc ccgcacgctg cgcccgcctt gggtgctgcg      720

ggcctgcctg gccccgggag cgcgctcaag ggcttggccg gtgcgcaccc cgacctccgc      780

acgggaggcg gcggcggtgg cagcggtgcc ggtgcgggca aagccaagaa gtcggtggac      840

aagaacagca acgagtaccg ggtacggcgg gaacgcaaca acatcgcggt gcgcaagagc      900

cgagataaag ccaaacaacg caacgtggag acgcaacaga aggtgctgga gttgaccagt      960

gacaatgacc gcctgcgcaa gcgggtggaa cagctgagcc gtgaactgga cacgctgcgg     1020

ggcatcttcc gccagctgcc tgagagctcc ttggtcaagg ccatgggcaa ctgcgcgtga     1080

<210>  20
<211>  359
<212>  PRT
<213>  Mus musculus

<400> 20

Met Glu Ser Ala Asp Phe Tyr Glu Val Glu Pro Arg Pro Pro Met Ser
1               5                   10                  15

Ser His Leu Gln Ser Pro Pro His Ala Pro Ser Asn Ala Ala Phe Gly
            20                  25                  30

Phe Pro Arg Gly Ala Gly Pro Ala Pro Pro Pro Ala Pro Pro Ala Ala
        35                  40                  45

Pro Glu Pro Leu Gly Gly Ile Cys Glu His Glu Thr Ser Ile Asp Ile
    50                  55                  60

Ser Ala Tyr Ile Asp Pro Ala Ala Phe Asn Asp Glu Phe Leu Ala Asp
65                  70                  75                  80

Leu Phe Gln His Ser Arg Gln Gln Glu Lys Ala Lys Ala Ala Ala Gly
                85                  90                  95

Pro Ala Gly Gly Gly Gly Asp Phe Asp Tyr Pro Gly Ala Pro Ala Gly
            100                 105                 110

Pro Gly Gly Ala Val Met Ser Ala Gly Ala His Gly Pro Pro Pro Gly
        115                 120                 125

Tyr Gly Cys Ala Ala Ala Gly Tyr Leu Asp Gly Arg Leu Glu Pro Leu
    130                 135                 140

Tyr Glu Arg Val Gly Ala Pro Ala Leu Arg Pro Leu Val Ile Lys Gln
145                 150                 155                 160

Glu Pro Arg Glu Glu Asp Glu Ala Lys Gln Leu Ala Leu Ala Gly Leu
                165                 170                 175

Phe Pro Tyr Gln Pro Pro Pro Pro Pro Pro Pro Pro His Pro His Ala
            180                 185                 190

Ser Pro Ala His Leu Ala Ala Pro His Leu Gln Phe Gln Ile Ala His
        195                 200                 205

Cys Gly Gln Thr Thr Met His Leu Gln Pro Gly His Pro Thr Pro Pro
    210                 215                 220

Pro Thr Pro Val Pro Ser Pro His Ala Ala Pro Ala Leu Gly Ala Ala
225                 230                 235                 240

Gly Leu Pro Gly Pro Gly Ser Ala Leu Lys Gly Leu Ala Gly Ala His
                245                 250                 255

Pro Asp Leu Arg Thr Gly Gly Gly Gly Gly Gly Ser Gly Ala Gly Ala
            260                 265                 270

Gly Lys Ala Lys Lys Ser Val Asp Lys Asn Ser Asn Glu Tyr Arg Val
        275                 280                 285

Arg Arg Glu Arg Asn Asn Ile Ala Val Arg Lys Ser Arg Asp Lys Ala
    290                 295                 300

Lys Gln Arg Asn Val Glu Thr Gln Gln Lys Val Leu Glu Leu Thr Ser
305                 310                 315                 320

Asp Asn Asp Arg Leu Arg Lys Arg Val Glu Gln Leu Ser Arg Glu Leu
                325                 330                 335

Asp Thr Leu Arg Gly Ile Phe Arg Gln Leu Pro Glu Ser Ser Leu Val
            340                 345                 350

Lys Ala Met Gly Asn Cys Ala
        355